# EUROPEAN PATENT SPECIFICATION

(11) **EP 2 279 245 B1**
(45) Date of publication and mention of the grant of the patent: **21.08.2013**
(21) Application number: 09735911.1
(22) Date of filing: 23.04.2009
(51) Int. Cl.: G01N 33/50, C12N 5/00

(54) **DRUG DISCOVERY METHODS INVOLVING A PRECLINICAL, IN VITRO ISOLATED GASTROINTESTINAL EPITHELIAL STEM CELL-LIKE PROGENITOR CELL SYSTEM**
WIRKSTOFFFINDUNGSVERFAHREN MIT EINEM VORKLINISCHEN IN-VITRO-SYSTEM MIT ISOLIERTEN MAGEN-DARM-EPITHEL-STAMMZELLEN-ÄHNLICHEN VORLÄUFERZELLEN
PROCÉDÉS DE RECHERCHE DE MÉDICAMENT IMPLIQUANT UN SYSTÈME DE CELLULE PROGÉNITRICE DE TYPE CELLULE SOUCHE ÉPITHÉLIALE GASTRO-INTESTINALE ISOLÉE IN VITRO, PRÉCLINIQUE

(30) Priority: 23.04.2008 US 47296 P
(43) Date of publication of application: 02.02.2011
(73) Proprietor: AlfaGene Bioscience, Inc., Somerset, NJ 08873 (US)
(72) Inventor: PANJA, Asit, Somerset, NJ 08873 (US)
(74) Representative: Denjean, Eric
(86) International application number: PCT/US2009/041549
(87) International publication number: WO 2009/132196

(56) References cited:
- WO-A2-02/057430
- US-A1- 2005 256 077
- US-A1- 2008 064 088
- ASIT PANJA: "A Novel Method for the Establishment of a Pure Population of Nontransformed Human Intestinal Primary Epithelial Cell (HIPEC) Lines in Long Term Culture", LABORATORY INVESTIGATION, vol. 80, no. 9, 1 September 2000 (2000-09-01), pages 1473-1475, XP55002003, ISSN: 0023-6837, DOI: 10.1038/labinvest.3780154
- SPRADLING ET AL.: 'Stem cells find their niche.' NATURE vol. 414, November 2001, pages 98 - 104, XP009078342
- BRITTAN ET AL.: 'Stem cell in gastrointestinal structure and neoplastic development.' GUT vol. 53, 2004, pages 899 - 910, XP008146410
- SQUIER ET AL.: 'Biology of oral mucosa and esophagus.' JOURNAL OF NATIONAL CANCER INSTITUTE MONOGRAPHS no. 29, 2001, pages 7 - 15, XP008146414
- VAN ES ET AL.: 'Wnt signalling induces maturation of paneth cells in intestinal crypts.' NATURE CELL BIOLOGY LETTERS 20 March 2005, pages 1 - 6, XP008146412
- HINNEBUSCH ET AL.: 'Enterocyte differentiation marker intestinal alkaline phosphatase is a target gene of the gut enriched Kruppel-like factor.' AM. J. PHYSIOL GASTROINTEST LIVER PHYSIOL vol. 286, 2004, pages G23 - G30, XP008146422
- RINDI ET AL.: 'Targeted ablation of secretin-producing cells in transgenic mice reveals a common differentiation pathway with multiple enteroendocrine cell lineages in the small intestine.' DEVELOPMENT vol. 126, 1999, pages 4149 - 4156, XP008146426

## Description

### FIELD OF THE INVENTION

The described invention relates to systems comprising isolated human gastrointestinal epithelial stem cell-like progenitor cells and uses of that system in drug discovery.

### BACKGROUND OF THE INVENTION

### Components of the Human Gastrointestinal Tract

The gastrointestinal tract is a continuous tube that extends from the mouth to the anus. On a gross level, the gastrointestinal tract is composed of the following organs: the mouth, most of the pharynx, the esophagus, the stomach, the small intestine (duodenum, jejunum and ileum), and the large intestine. Each segment of the gastrointestinal tract participates in the absorptive processes essential to digestion by producing chemical substances that facilitate digestion of orally taken foods, liquids, and other substances such as therapeutic agents.

Within the gastrointestinal tract, the small intestine, the site of most digestion and absorption, is structured specifically for these important functions. The small intestine is divided into three segments: the duodenum, the jejunum, and the ileum. The absorptive cells of the small intestine produce several digestive enzymes called the 'brush-border' enzymes. Together with pancreatic and intestinal juices, these enzymes facilitate the absorption of substances from the chime in the small intestine. The large intestine, the terminal portion of the gastrointestinal tract, contributes to the completion of absorption, the production of certain vitamins, and the formation and expulsion of feces.

At the cellular level, the epithelium is a purely cellular avascular tissue layer that covers all free surfaces (cutaneous, mucous, and serous) of the body including the glands and other structures derived from it. It lines both the exterior of the body, as skin, and the interior cavities and lumen of the body. While the outermost layer of human skin is composed of dead stratified squamous, keratinized epithelial cells, mucous membranes lining the inside of the mouth, the esophagus, and parts of the rectum are themselves lined by nonkeratinized stratified squamous epithelium. Epithelial cell lines are present inside of the lungs, the gastrointestinal tract, and the reproductive and urinary tracts, and form the exocrine and endrocrine glands.

Epithelial cells are involved in secretion, absorption, protection, transcellular transport, sensation detection and selective permeability. There are variations in the cellular structures and functions in the epithelium throughout the gastrointestinal tract. The epithelium in the mouth, pharynx, esophagus and anal canal is mainly a protective, nonkeratinized, squamous epithelium. The epithelium of the stomach is composed of (i) simple columnar cells that participate in nutrient and fluid absorption and secretion, (ii) mucus producing goblet cells that participate in protective and mechanical functions, and (iii) enteroendocrine cells that participate in the secretion of gastrointestinal hormones. Additionally, within the intestine, the epithelial lining provides an important defense barrier against microbial pathogens.

The development of intestinal epithelium involves three major phases: 1) an early phase of epithelial proliferation and morphogenesis; 2) an intermediate period of cellular differentiation in which the distinctive cell types characteristic of intestinal epithelium appear; and 3) a final phase of biochemical and functional maturation. Intestinal crypts, located at the base of villi, contain stem cells which supply the entire epithelial cell surface with a variety of epithelial cell subtypes. These specialized cells provide for an external environment-internal environment interface, ion and fluid secretion and reabsorption, antigen recognition, hormone secretion, and surface protection. The exposure of epithelial cells on the surfaces of the intestinal lumen subjects them to a wide range of assaults, including microbial, chemical, and physical forces; thus they also may contribute to patho-physiologic impairment in diseases. Additionally, these cells are targets for inflammation, infection, and malignant transformation.

Within the intestinal tract, the epithelium forms upon stem cell differentiation. Stem cells are undifferentiated cells having high proliferative potential with the ability to self-renew that may generate daughter cells that may undergo terminal differentiation into more than one distinct cell type. A cell that is able to differentiate into many cell types may be referred to as "pluripotent." A cell that is able to differentiate into all cell types may be referred to as "totipotent." Pluripotent stem cells undergo further specialization into multipotent progenitor cells that then give rise to functional cells. For example, hematopoietic stem cells give rise to red blood cells, white blood cells, and platelets. Mesenchymal stem cells are multipotent cells that are capable of differentiating along several lineage pathways, including, but not limited to, chondrocytes, osteoblasts, adipocytes, fibroblasts, marrow stroma, and other tissues of mesenchymal origin. Epithelial stem cells give rise to the various types of skin cells; and muscle satellite cells contribute to differentiated muscle tissue. The technologies for retrieval, and maintenance of epithelial stem cells in an undifferentiated state, and for growing them *in vitro* have been the subject of study.

### Molecular Markers of Gastrointestinal Epithelial Stem Cells

Specialized protein receptors that have the capability to selectively bind or adhere to other signaling molecules coat the surface of every cell in the body. Cells use these receptors and the molecules that bind to them as a way of communicating with other cells and to carry out their proper functions in the body. Each cell type has a certain combination of receptors, or markers, on their surface that makes them distinguishable from other kinds of cells.

Stem cell markers are given short-hand names based on the molecules that bind to the corresponding stem cell surface receptors. In many cases, a combination of multiple markers is used to identify a particular stem cell type. Researchers often identify stem cells in shorthand by a combination of marker names reflecting the presence (+) or absence (-) of them. For example, a special type of hematopoietic stem cell from blood and bone marrow called "side population" or "SP" is described as (CD34-/low, c-Kit+, Sca-1+).

The following markers commonly are used by skilled artisans to identify stem cells and to characterize differentiated cell types:

| **Marker** | **Cell Type** | **Significance** |
|---|---|---|
| CD34 | Hematopoietic stem cell (HSC), muscle satellite, endothelial progenitor | A highly glycosylated type I transmembrane protein expressed on 1-4% of bone marrow cells |
| CD38 | immature T and B cells | A type II transmembrane protein found on immature T and B cells but not most mature peripheral lymphocytes |
| CD41 | platelets and megakaryocytes | The integrin αIIb subunit |
| CD45 | WBC progenitor | The leukocyte common antigen found on all cells of hematopoietic origin |
| CD105 | Endothelial cells | A disulfide-linked homodimer found on endothelial cells but absent from most T and B cells |
| CD133 | primitive hematopoietic progenitors | A pentaspan transmembrane glycoprotein |
| CD3 | T cells | A member of the T cell receptor complex |
| CD4, CD8 | Mature T cells | Cell-surface protein markers specific for mature T lymphocyte (WBC subtype) |
| CD7 | Early T cells | An early T cell lineage marker |
| CD10 | early T and B cell precursors | A type II membrane metalloprotease |
| CD13 | granulocytes, monocytes and | A type II membrane metalloprotease |
| | their precursors | |
| CD14 | myelomonocytic lineage | A GPI-linked protein expressed mainly on myelomonocytic lineage cells |
| CD19 | B cells | A component of the B cell antigen signaling complex |
| CD33 | Myelomonocytic precursors | A sialic acid binding protein absent from pluripotent stem cells that appears on myelomonocytic precursors after CD34 |
| CD38 | WBC lineages | A cell-surface molecule that identifies WBC lineages. Selection of CD34+/CD38- cells allows for purification of HSC populations |
| CD44 | Mesenchymal | A type of cell-adhesion molecule used to identify specific types of mesenchymal cells |
| CD56 | NK cells | An isoform of the neural adhesion molecule found exclusively on natural killer (NK) cells; |
| CD127 | lymphocytes | The high affinity interleukin 7 receptor expressed on lymphocytes |
| CD138 | Immature B cells and plasma cells | An extracellular matrix receptor found on immature B cells and plasma cells |
| Glycophorin A | RBCs, embryoid precursors | A sialoprotein present on human RBCs and embryoid precursors |
| CD90 | prothymocytes | A GPI-cell anchored molecule found on prothymocyte cells in humans |
| c-kit | HSC, MSC | Cell-surface receptor on BM cell types that identifies HSC and MSC; binding by fetal calf serum (FCS) enhances proliferation of ES cells, HSCs, MSCs, and hematopoietic progenitor cells |
| Fetal liver kinase-1 (Flk-1) | endothelial | Cell-surface receptor protein that identifies endothelial cell progenitor; marker of cell-cell contacts |

To our knowledge, there are no universally accepted molecular markers that identify gastrointestinal stem cells. However, several markers have been used to identify stem cells in small and large intestinal tissues. These include: β-1-integrin, mushashi-1, CD45, and cytokeratin.

CD45, also called the common leukocyte antigen, T220 and B220 in mice, is a transmembrane protein with cytoplasmic protein tyrosine phosphatase (PTP) activity. CD45 is found in hematopoietic cells except erythrocytes and platelets. CD45 has several isoforms that can be seen in the various stages of differentiation of normal hematopoietic cells.

Mushashi-1 is an early developmental antigenic marker of stem cells and glial/neuronal cell precursor cells.

β-1-integrin (CD29, fibronectin receptor), is a β-subunit of a heterodimer protein member of the integrin family of proteins; integrins are membrane receptors involved in cell adhesion and recognition.

Cytokeratins are intermediate filament proteins found in the intracytoplasmic cystoskeleton of the cells that comprise epithelial tissue.

There are four main epithelial cell lineages: (i) columnar epithelial cells, (ii) goblet cells, (iii) enteroendocrine chromaffin cells, and (iv) Paneth cells. Several molecular markers have been used to identify each of these lineages.

The markers used to identify columnar epithelial cells include: intestinal alkaline phosphatase (ALP1), sucrase isomaltase (SI), sodium/glucose cotransporter (SLGT1), dipeptidyl-peptidase 4 (DPP4), and CD26. Intestinal alkaline phosphatase (E.C. 3.1.3.1) is a membrane-bound enzyme localized in the brush border of enterocytes in the human intestinal epithelium. Sucrase-isomaltase (SI, EC 3.2.1.48) is an enterocyte-specific small intestine brush-border membrane disaccharidase. Dipeptidyl-peptidase 4 (E.C. 3.4.14.5) is a membrane bound serine-type peptidase. Sodium/glucose transporter (SGLT) mediates transport of glucose into epithelial cells. SGLT belongs to the sodium/glucose cotransporter family SLCA5. Two different SGLT isoforms, SGLT1 and SGLT2, mediate renal tubular glucose reabsorption in humans. Both of them are characterized by their different substrate affinity. SGLT1 transports glucose as well as galactose, and is expressed both in the kidney and in the intestine. SGLT2 transports glucose and is believed to be responsible for 98% of glucose reabsorption; SGLT2 is generally found in the S1 and S2 segments of the proximal tubule of the nephron. CD26 is a multifunctional protein of 110 KDa strongly expressed on epithelial cells (kidney proximal tubules, intestine, and bile duct) and on several types of endothelial cells and fibroblasts and on leukocyte subsets.

The markers used to identify goblet cells include mucin 2 (MUC2) and trefoil factor 3 (TFF3). Mucin-2, a secreted gel-forming mucin, is the major gel-forming mucin secreted by goblet cells of the small and large intestines and is the main structural component of the mucus gel. Intestinal trefoil factor 3 is a nonmucin protein and a product of fully differentiated goblet cells.

The markers used to identify enteroendocrine chromaffin cells include chromogranin A (CHGA) and synaptophysin (SYP). Chromogranin A (CHGA) and its derived peptides, which are stored and released from dense-core secretory granules of neuroendocrine cells, have been implicated as playing multiple roles in the endocrine, cardiovascular, and nervous systems. Synaptophysin I (SYP) is a synaptic vesicle membrane protein that is ubiquitously expressed throughout the brain without a definite synaptic function.

The markers used to identify Paneth cells include lysozyme (LYZ), defensin (DEFA1), and matrix metallopeptidase 7 (MMP7). Lysozyme (LYZ or muramidase) (E.C. 3.2.1.17) catalyzes the hydrolysis of 1,4-beta-linkages between N-acetylmuramic acid and N-acetyl-D-glucosamine residues in a peptidoglycan and between N-acetyl-D-glucosamine residues in chitodextrins. Defensins (DEFA1) are small peptides that are produced by leukocytes and epithelial cells. Human defensin α-1 is a 3.5-kDa, 30-amino-acid peptide that has shown effector functions in host innate immunity against some microorganisms. Matrix metalloproteinases (MMPs) are a family of metal-dependant enzymes that are responsible for the degradation of extracellular matrix components. MMPs are involved in various physiologic processes, such as embryogenesis and tissue remodeling and also play a role in invasion and metastasis of tumor cells, which require proteolysis of basal membranes and extracellular matrix.

### Considerations That Affect Gastrointestinal Absorption Of Therapeutic Agents

Gastrointestinal absorption is important to the bioavailability and therapeutic effectiveness of an orally administered therapeutic agent.

Oral ingestion is the most common route of therapeutic agent administration. A therapeutic agent administered orally is absorbed and transferred by the gastrointestinal epithelium to the site of action of that agent. This transfer process subjects the therapeutic agent to structural alterations and potential changes in pharmacological properties. As a result, a therapeutic agent identified as having potentially beneficial therapeutic effect by preclinical studies may lose its biological activity or become toxic. For example, an orally administered therapeutic agent must be absorbed from the stomach and intestine. This absorption may be limited by the characteristics of the dosage form and/or the therapeutic agent's physiochemical properties. The therapeutic agent then passes through the liver, where metabolism and/or biliary excretion may occur before it reaches the systemic circulation. Accordingly, a fraction of the administered and absorbed dose of drug will be inactivated or diverted before it can reach the general circulation and be distributed to its sites of action. If the metabolic or excretory capacity of the liver for the therapeutic agent in question is large, bioavailability will be substantially reduced (*i.e*., the first-pass effect). This decrease in availability is a function of the anatomical site from which absorption takes place; other anatomical, physiological, and pathological factors also can influence bioavailability.

The gastrointestinal permeability of therapeutic agents that can be administered orally is evaluated as a part of the general discovery and development process. Although the rate of therapeutic agent absorption does not, in general, influence the average steady-state concentration of therapeutic agent in plasma, it may influence therapeutic therapy. If a therapeutic agent is absorbed rapidly (*e.g*., a dose given as an intravenous bolus) and has a small "central" volume, the concentration of therapeutic agent initially will be high. It will fall as the therapeutic agent is distributed to its "final" (*i.e*., larger) volume. If the same therapeutic agent is absorbed more slowly (*e.g*. ,a dose given by slow infusion), it will be distributed while it is being given, and peak concentrations will be lower and will occur later. Controlled-release preparations are designed to provide a slow and sustained rate of absorption in order to produce a less fluctuating plasma concentration-time profile during the dosage interval compared to more immediate-release formulations. A given drug may act to produce both desirable and undesirable effects at several sites in the body, and the rates of distribution of therapeutic agent to these sites may not be the same. The relative intensities of these different effects of a therapeutic agent thus may vary transiently when the rate of administration is changed.

Absorption from the gastrointestinal tract is governed by such factors as surface area available for absorption, blood flow to the site of absorption, the physical state of the therapeutic agent (*e.g*., solution, suspension, or solid dosage form), its water solubility, and its concentration at the site of absorption. For therapeutic agents given in solid form, the rate of dissolution may be the limiting factor in their absorption, especially if they have low water solubility.

Since most absorption from the gastrointestinal tract occurs *via* passive processes, absorption is favored when the therapeutic agent is in the nonionized and more lipophilic form. Most therapeutic agents are weak acids or bases that are present in solution as both the nonionized and ionized species. The nonionized molecules are usually lipid-soluble and may diffuse across the cell membrane. In contrast, the ionized molecules are usually unable to penetrate the lipid membrane because of their low lipid solubility. Therefore, the transmembrane distribution of a weak electrolyte usually is determined by its pK*ₐ* and the pH gradient across the membrane, wherein the pK*ₐ* is the pH at which half of the weak electrolyte is in its ionized form. The gastric mucosal membrane may be assumed to behave as a simple lipid barrier that is permeable only to the lipid-soluble, nonionized form of the acid. The ratio of nonionized to ionized therapeutic agent at each pH is calculated readily from the Henderson-Hasselbalch equation. Thus, in plasma, the ratio of nonionized to ionized therapeutic agent is 1:1000; in gastric juice, the ratio is 1:0.001. The total concentration ratio between the plasma and the gastric juice would therefore be 1000:1 if such a system came to a steady state. For a weak base with a pK*ₐ* of 4.4, the ratio would be reversed. Accordingly, at steady state, an acidic therapeutic agent would accumulate on the more basic side of the membrane and a basic therapeutic agent on the more acidic side. This phenomenon is referred to as "ion trapping." These considerations have implications on absorption and excretion of therapeutic agents. The establishment of concentration gradients of weak electrolytes across membranes with a pH gradient is purely a physical process and does not require an active transport system. All that is necessary is a membrane preferentially permeable to one form of the weak electrolyte and a pH gradient across the membrane. The establishment of the pH gradient is, however, an active process. Based on the pH-partition concept, it would be predicted that therapeutic agents that are weak acids would be better absorbed from the stomach (pH 1 to 2) than from the upper intestine (pH 3 to 6), and *vice versa* for weak bases. However, the epithelium of the stomach is lined with a thick mucous layer, and its surface area is small; by contrast, the villi of the upper intestine provide an extremely large surface area (approximately 200 m²). Accordingly, the rate of absorption of a therapeutic agent from the intestine is greater than that from the stomach even if the therapeutic agent is predominantly ionized in the intestine and largely nonionized in the stomach. Thus, any factor that accelerates gastric emptying will be likely to increase the rate of therapeutic agent absorption, while any factor that delays gastric emptying probably will have the opposite effect, regardless of the characteristics of the therapeutic agent.

Therapeutic agents that are destroyed by gastric juice or that cause gastric irritation sometimes may be administered in dosage forms with a coating that prevents dissolution in the acidic gastric contents. However, some enteric-coated preparations of a therapeutic agent also may resist dissolution in the intestine, and very little of the therapeutic agent may be absorbed.

The rate of absorption of a therapeutic agent administered as a tablet or other solid oral-dosage form is partly dependent upon its rate of dissolution in the gastrointestinal fluids. This factor is the basis for the controlled-release, extended-release, sustained-release, or prolonged-action pharmaceutical preparations that are designed to produce slow, uniform absorption of the drug for 8 hours or longer. Potential advantages of such preparations are (i) reduction in the frequency of administration of the therapeutic agent as compared with conventional dosage forms (possibly with improved compliance by the patient), (ii) maintenance of a therapeutic effect overnight, and (iii) decreased incidence and/or intensity of undesired effects by elimination of the peaks in drug concentration that often occur alter administration of immediate-release dosage forms.

### Available Models are Insufficient

Document US 2005/256077 relates to the isolation of a special population of cells by flow cytometry from mouse tissue and identifies them as stem cells.
Document Panja (Lab. Invest. 2000. 80:9; 1473-1475) discloses a method to generate a pure population of non transformed human intestinal epithelial cell lines (HIPEC), cultured on collagen-coated petri dishes, in the presence of mucosal tissue-derived growth factor containing culture supernatants and a combination of hormonal supplements.
Document WO 02/057430 discloses a method for isolating stem cell populations, for example intestinal stem cells, and cultivating them on a matrix comprising fibroblast feeder cells isolated from human intestine.

It has proved difficult to develop in vitro systems that reflect physiological conditions within the human gastrointestinal tract.

Because systems and methods that simultaneously provide adult intestinal epithelial cells that can reproducibly survive in culture and that have normal functional characteristics are lacking, there is a need in the art for an adequate preclinical model that provides an environment similar to the actual physiological environment of a human gastrointestinal tract.

The described invention provides a preclinical, in vitro system comprising gastrointestinal epithelial stem cell-like progenitor cells having structural and functional characteristics of the normal human gastrointestinal tract and methods for the development of therapeutics using these systems.

### SUMMARY OF THE INVENTION

The described invention relates to systems comprising isolated human gastrointestinal epithelial stem cell-like progenitor cells and uses of that system in drug discovery.

According to one aspect, the described invention provides a system to determine the bioavailability of a therapeutic agent, **in at least one gastrointestinal segment of a human**, comprising differentiable gastrointestinal segment-specific human epithelial stem-cell-like progenitor cells isolated from at least one human mucosal tissue derived from at least one human gastrointestinal segment **and a growth substrate comprising a segment specific bio-similar matrix environment formed from the segment of the gastrointestinal human mucosal tissue.** According to one embodiment, the gastrointestinal segment-specific human epithelial stem cell-like progenitor cells are cultivated **thereon**. According to another embodiment, the segment is a stomach segment. According to another embodiment, the segment is a jejunum segment. According to another embodiment, the segment is an ileum segment. According to another embodiment, the segment is a duodenum segment. According to another embodiment, the segment is an ascending colon segment. According to another embodiment, the segment is a transverse colon segment. According to another embodiment, the segment is a sigmoid colon segment. According to another embodiment, the segment is a rectum segment. According to another embodiment, the differentiable gastrointestinal segment-specific human epithelial stem-cell-like progenitor cell optionally differentiates into a mature cell phenotype. According to another embodiment, the mature cell phenotype is a columnar epithelial cell. According to another embodiment, the mature cell phenotype is a Paneth cell. According to another embodiment, the mature cell phenotype is a goblet cell. According to another embodiment, the mature cell phenotype is an enteroendocrine chromaffin cell. According to another embodiment, the mature cell phenotype is a neuronal cell type. According to another embodiment, the differentiable gastrointestinal segment-specific human epithelial stem-cell-like progenitor cell is a mesenchymal cell. According to another embodiment, the system is used to assess at least one parameter of permeability of the therapeutic agent. According to another embodiment, the system is used to assess absorption of the therapeutic agent. According to another embodiment, the system is used to assess uptake of the therapeutic agent. According to another embodiment, the system is used to assess cellular toxicity of the therapeutic agent. According to another embodiment, the system is used to assess transepithelial electrical resistance. According to another embodiment, the differentiable gastrointestinal segment-specific human stem epithelial cell-like progenitor cells on the at least one bio-similar matrix environment are used to determine variations in DNA and/or RNA characteristics produced in response to the therapeutic agent. According to another embodiment, the system is used to determine segment-specific metabolic byproducts of the therapeutic agent. According to another embodiment, the bio-similar matrix environment formed from the at least one mucosal tissue derived from the stomach is serially connected to the bio-similar matrix environment formed from the at least one mucosal tissue derived from the jejunum, which is serially connected to the bio-similar matrix environment formed from the at least one mucosal tissue derived from the ileum, which is serially connected to the bio-similar matrix environment formed from the at least one mucosal tissue derived from the duodenum, which is serially connected to the bio-similar matrix environment formed from the at least one mucosal tissue derived from the ascending colon segment, which is serially connected to the bio-similar matrix environment formed from the at least one mucosal tissue derived from the transverse colon segment, which is serially connected to the bio-similar matrix environment formed from the at least one mucosal tissue derived from the sigmoid colon segment, which is serially connected to the bio-similar matrix environment formed from the at least one mucosal tissue derived from the rectum to form an *in vitro* model of the human gastrointestinal tract. According to another embodiment, the differentiable gastrointestinal segment-specific human epithelial stem cell-like progenitor cell has at least a β-1-integrin⁽⁺⁾cytokeratin⁽⁺⁾ phenotype. According to another embodiment, the differentiable gastrointestinal segment-specific human epithelial stem cell-like progenitor cell has a phenotype of cytokeratin(+), β-1-integrin(+), defensin-5(+), trefoil factor-3(+), mucin-2(+), chomogranin-A(+), intestinal alkaline phosphatase(+), lysozyme(+).

According to another aspect, the described invention provides a method to determine gastrointestinal segmental effectiveness of a therapeutic agent, the method comprising the steps: (a) isolating differentiable gastrointestinal segment-specific human epithelial stem-cell-like progenitor cells from at least one mucosal tissue derived from at least one human gastrointestinal segment; (b) cultivating the differentiable gastrointestinal segment-specific human epithelial stem cell-like progenitor cells on at least one bio-similar matrix environment formed from the at least one mucosal tissue derived from the at least one human gastrointestinal segment; (c) exposing the differentiable gastrointestinal segment-specific human epithelial stem cell-like progenitor cells on the at least one bio-similar matrix environment to the therapeutic agent; and (d) analyzing the differentiable gastrointestinal segment-specific human epithelial stem cell-like progenitor cells to determine regional specificity of the therapeutic agent. According to one embodiment, a first human gastrointestinal segment is a stomach segment. According to another embodiment, a first human gastrointestinal segment is a jejunum segment. According to another embodiment, a first human gastrointestinal segment is an ileum segment. According to another embodiment, a first human gastrointestinal segment is a duodenum segment. According to another embodiment, a first human gastrointestinal segment is an ascending colon segment. According to another embodiment, a first human gastrointestinal segment is a transverse colon segment. According to another embodiment, a first human gastrointestinal segment is a sigmoid colon segment. According to another embodiment, a first human gastrointestinal segment is a rectum segment. According to another embodiment, the differentiable gastrointestinal segment-specific human epithelial stem cell-like progenitor cells on the at least one bio-similar matrix environment are used to determine variations in DNA and/or RNA characteristics produced in response to the therapeutic agent. According to another embodiment, the method further comprises the step of serially connecting the bio-similar matrix environment formed from the at least one mucosal tissue derived from the stomach segment to the bio-similar matrix environment formed from the at least one mucosal tissue derived from the jejunum segment to the bio-similar matrix environment formed from the at least mucosal tissue derived from the ileum segment, to the bio-similar matrix environment formed from the at least one mucosal tissue derived from the duodenum segment to the bio-similar matrix environment formed from the at least one mucosal tissue derived from the ascending colon segment to the bio-similar matrix environment formed from the at least one mucosal tissue derived from the transverse colon segment to the bio-similar matrix environment formed from the at least one mucosal tissue derived from the sigmoid colon segment to the bio-similar matrix environment formed from the at least one mucosal tissue derived from the rectum to form an *in vitro* model of the human gastrointestinal tract. According to another embodiment, the differentiable gastrointestinal segment-specific human epithelial stem cell-like progenitor cell has at least a β-1-integrin⁽⁺⁾cytokeratin⁽⁺⁾ phenotype. According to another embodiment, the differentiable gastrointestinal segment-specific human stem epithelial cell-like progenitor cell has a phenotype of cytokeratin(+), β-1-integrin(+), defensin-5(+), trefoil factor-3(+), mucin-2(+), chomogranin-A(+), intestinal alkaline phosphatase(+), lysozyme(+).

According to another aspect, the described invention provides a method to identify therapeutic targets useful in treating inflammatory diseases of the gastrointestinal tract, the method comprising the steps: (a) isolating differentiable gastrointestinal segment-specific human epithelial stem-cell-like progenitor cells from at least one human mucosal tissue derived from at least one human gastrointestinal segment; (b) cultivating the differentiable gastrointestinal segment-specific human epithelial stem cell-like progenitor cells on at least one bio-similar matrix environment formed from the at least one human mucosal tissue derived from the at least one human gastrointestinal segment; (c) exposing the differentiable gastrointestinal segment-specific human epithelial stem cell-like progenitor cells on the at least one bio-similar matrix environment to a therapeutic agent; (d) analyzing the differentiable gastrointestinal segment-specific human epithelial stem cell-like progenitor cells on the at least one bio-similar matrix environment exposed to the therapeutic agent to identify at least one marker as a therapeutic target. According to one embodiment, a first human gastrointestinal segment is a stomach segment. According to another embodiment, a first human gastrointestinal segment is a jejunum segment. According to another embodiment, a first human gastrointestinal segment is an ileum segment. According to another embodiment, a first human gastrointestinal segment is a duodenum segment. According to another embodiment, a first human gastrointestinal segment is an ascending colon segment. According to another embodiment, a first human gastrointestinal segment is a transverse colon segment. According to another embodiment, a first human gastrointestinal segment is a sigmoid colon segment. According to another embodiment, a first human gastrointestinal segment is a rectum segment. According to another embodiment, the differentiable gastrointestinal segment-specific human epithelial stem cell-like progenitor cells on the at least one bio-similar matrix environment are used to determine variations in DNA and/or RNA characteristics produced in response to the therapeutic agent. According to another embodiment, the method further comprises the steps of between step (b) and step (c), serially connecting the bio-similar matrix environment formed from the at least one mucosal tissue derived from the stomach segment to the bio-similar matrix environment formed from the at least one mucosal tissue derived from the jejunum segment to the bio-similar matrix environment formed from the at least one mucosal tissue derived from the ileum segment to the bio-similar matrix environment formed from the at least one mucosal tissue derived from the to the bio-similar matrix environment formed from the at least one mucosal tissue derived from the duodenum segment to the bio-similar matrix environment formed from the at least one mucosal tissue derived from the ascending colon segment to the bio-similar matrix environment formed from the at least one mucosal tissue derived from the transverse colon segment to the bio-similar matrix environment formed from the at least one mucosal tissue derived from the sigmoid colon segment to the bio-similar matrix environment formed from the at least one mucosal tissue derived from the rectum to form an *in vitro* model of the human gastrointestinal tract; and in step (c) of the method, serially administering the therapeutic agent to the differentiable gastrointestinal segment-specific human stem-cell-like progenitor cells isolated from a human mucosal tissue derived from a human gastrointestinal segment on the serially connected biosimilar matrix environments. According to another embodiment, the differentiable gastrointestinal segment-specific human epithelial stem cell-like progenitor cell has at least a β-1-integrin⁽⁺⁾cytokeratin⁽⁺⁾ phenotype. According to another embodiment, the differentiable gastrointestinal segment-specific human epithelial stem cell-like progenitor cell has a phenotype of cytokeratin(+), β-1-integrin(+), defensin-5(+), trefoil factor-3(+), mucin-2(+), chomogranin-A(+), intestinal alkaline phosphatase(+), lysozyme(+).

### BRIEF DESCRIPTION OF THE DRAWINGS

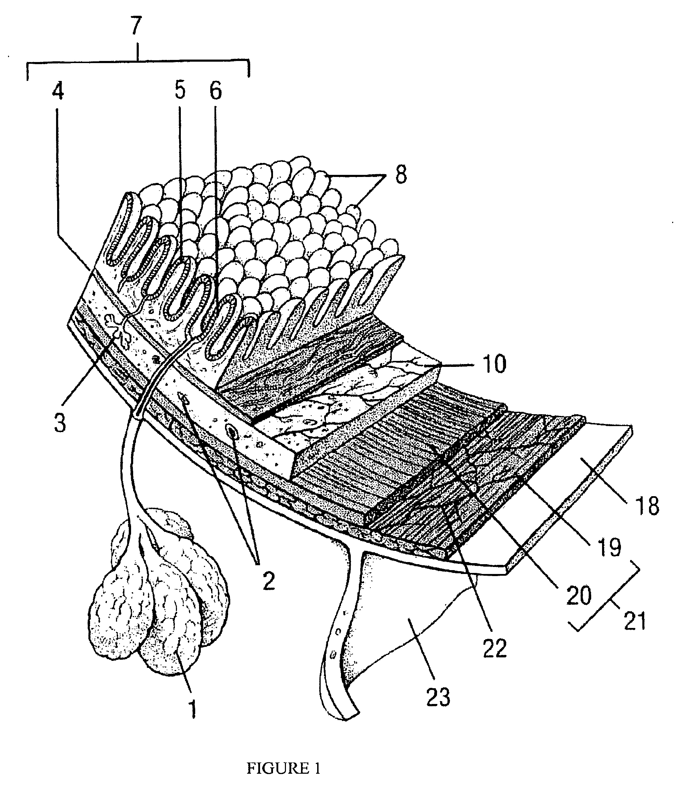

**Figure 1** shows a diagram of the four main layers of the wall of the human intestine: 1) gland outside gut but developing from it (liver); 2) blood vessels; 3) gland in submucosa; 4) muscularis mucosae; 5) epithelium; 6) lamina propria; 7) mucous membrane; 8) villi; 10) submucosa; 18) serosa; 19) circular muscle; 20) longitudinal muscle; 21) muscularis; 22) myenteric plexus; and 23) mesentery. (©2000, Stedman's Medical Dictionary, 27th Ed, Lippincott Williams & Wilkins, Baltimore, MD. p.915).
**Figure 2** shows a light photomicrograph of one embodiment of a cell monolayer of an intestinal (jejunal) stem-cell-like progenitor cell population grown on mucosal derived matrix coated plastic surface in culture.
**Figure 3** shows an agaroase gel containing PCR amplification products from one embodiment of a primary cell culture derived from isolated human gastrointestinal epithelial stem-cell-like progenitor crypt cells from small and large intestinal segments.
**Figure 4** shows a light photomicrograph of cells stained with anti-cytokeratin-18 antibody.
**Figure 5** shows light photomicrographs of one embodiment of primary epithelial monolayers derived from human gastrointestinal stem-cell-like progenitor cells isolated from various segments of the human gastrointestinal tract. A) duodenum; B) jejunum; C) ascending colon; D) transverse colon; E) sigmoid; and F) rectum. All colonic HIPEC lines were derived from the normal portion of a resected specimen from patients suffering different disorders.
**Figure 6** shows a photograph (10X magnification) of HIPEC lines grown on soft agar, and stained with 0.1 % crystal violet, destained. A) and C) normal colonic HIPEC line; B) and D) control malignant colonic epithelial cell line HT29. No growth is observed on A) and C); foci formation and cell growth observed on B) and D).
**Figure 7** shows the karyotype of a nontransformed HIPEC line. Karyotype analysis of metaphase chromosomes of one embodiment of a cell showed a normal 46 XY complement.
**Figure 8** shows a plot of fluorescence intensity versus cell number of HIPEC lines expressing intestine specific enzyme markers intestinal alkaline phosphatase (IAP) and sucrase isomaltase (SI). Immunofluorescence staining of representative HIPEC lines from small (jejunum (left panel)) and large (colon (right panel)) intestine with anti-human intestinal alkaline phosphatase (upper panel) and sucrase isomaltase (lower panel).
**Figure 9** shows an electron microscope image (1000X magnification) of ultrastructures within one embodiment of a HIPEC line at passage 4. Microvilli (M) at the apical surface, large nuclei and bundles of microfilaments, and intercellular tight junction (TJ) are present.

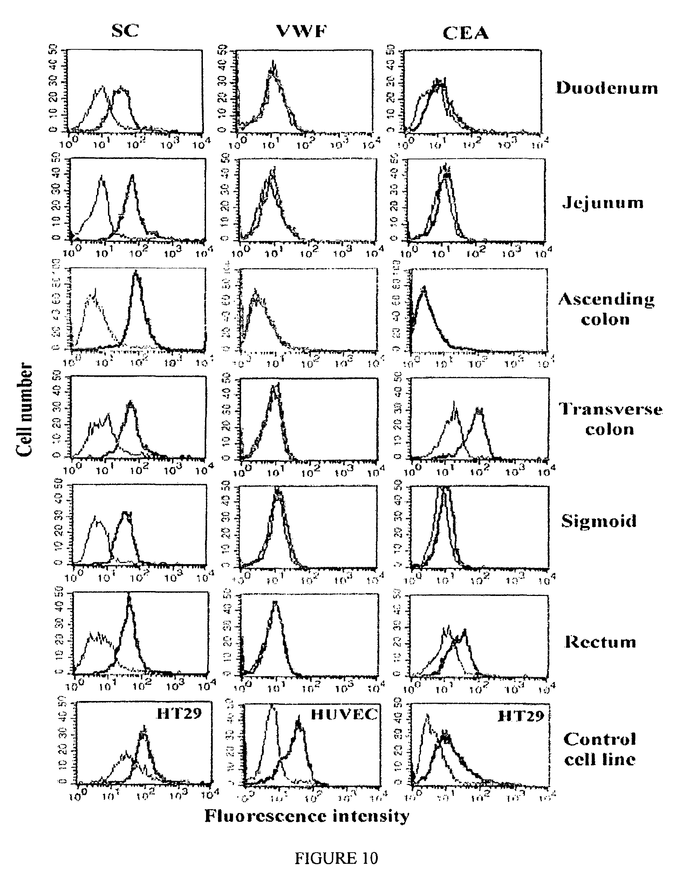

**Figure 10** shows plots of the flowcytometric analysis of the cells stained with an anti-CEA (right panel - red lines), anti-VWF (middle panel - green lines), anti-SC (left panel - blue lines), or control antibody (black lines). For the detection of SC, staining was performed on unpermeabilized cells. All HIPEC lines were positive for secretory component (left panel) and negative for VWF (middle panel) and SMC (data not shown). Variable level of CEA expression (middle panel) was observed. The bottom panel row are control cell lines: the colonic adenocarcinoma cell line HT29 used to demonstrate both SC (left panel) and CEA (right panel) expression and a endothelial cell line, HUVEC (center panel) used to demonstrate VWF expression.

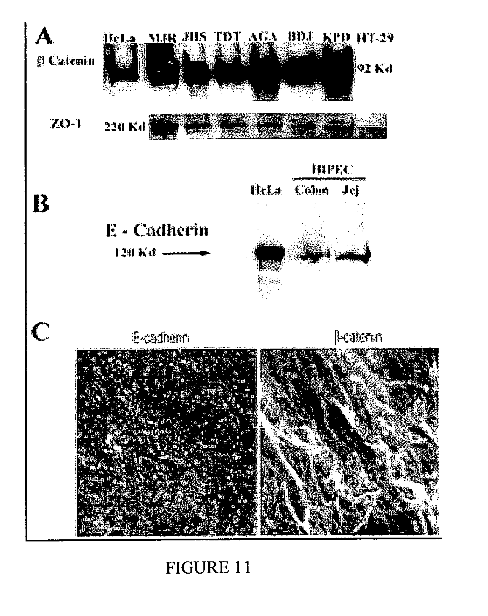

**Figure 11** shows junction protein expression in HIPEC monolayers. (A) Western blot analysis of lysates from HIPEC lines representing various segments of the intestine (MJR = rectum, JHS = sigmoid, TDT = transverse colon, AGA = ascending colon, BDJ = jejunum, KPD = duodenum) using anti-β-catenin (upper panel) and anti-ZO-1 (lower panel) antibodies. HeLa (first lane - upper panel), a uterine-derived malignant epithelial cell line, served as a positive control for β-catenin, and HT29 (last lane - lower panel), a colonic adenocarcinoma epithelial cell line, served as a positive control for ZO-1. All HIPEC lines expressed both β-catenin and ZO-1 (β-catenin>>>ZO-1). (**B**) Western blot analysis of lysates from one embodiment of HIPEC lines derived from a normal colon (WT) and small intestine (Jej) (BDJ) using an anti E-Cadherin monoclonal antibody. A lung tumor epithelial cell line A431 served as a positive control. (C) Immunohistochemical analysis of E-cadherin and β-catenin expression on one embodiment of a HIPEC line (on cover slips).
**Figure 12** shows a plot of transepithelial resistance (ohms/cm²) versus days in culture. The error bars represent standard deviation from the mean, n = 3 (performed in triplicate). Cells in culture are representative HGISC-derived epithelial cells from jejunum (A2J1) and colon (5A).
**Figure 13** shows a bar graph illustrating the amount of drug (naproxan) (µM) absorbed or unabsorbed through epithelial monolayers derived from human GI stem cells.

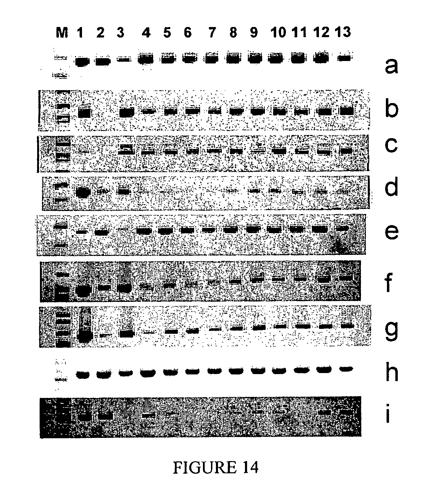

**Figure 14** shows agarose gels of the stem cell marker amplification products acquired from RT-PCR. Lane: (M) molecular weight markers (1) stem cell line control; (2) human dermal fibroblast cell line control; (3) peripheral blood monocyte control; (4) esophagus derived stem cell line D1708E; (5) gastric derived stem cell line D1708G; (6) duodenum derived stem cell line D1708D; (7) jejunum derived stem cell line D1708J; (8) ileum derived stem cell line D1708I; (9) cecum derived stem cell line D1708C; (10) ascending colon derived stem cell line D1708A; (11) transverse colon derived stem cell line D1708T; (12) sigmoid derived stem cell line D1708S; (13) rectum derived stem cell line D1708R. Stem cell markers: (a) β-tublin (385 bp); (b) Nanog (852 bp); (c) LIN28 (829 bp); (d) Oct4 (variant 1 and 2; 455 bp); (e) Oct4 (variant 2; 471 bp); (f) Oct4 (variant 1; 828 bp); (g) SOX2 (581 bp); (h) Bmi1 (576 bp); (i) Lgr5 (498 bp).
**Figure 15** shows agarose gels of the biomarker Oct4, Nanog and β-tubulin amplification products acquired during each of the 10 serial passages of the A2J1 stem cell line.

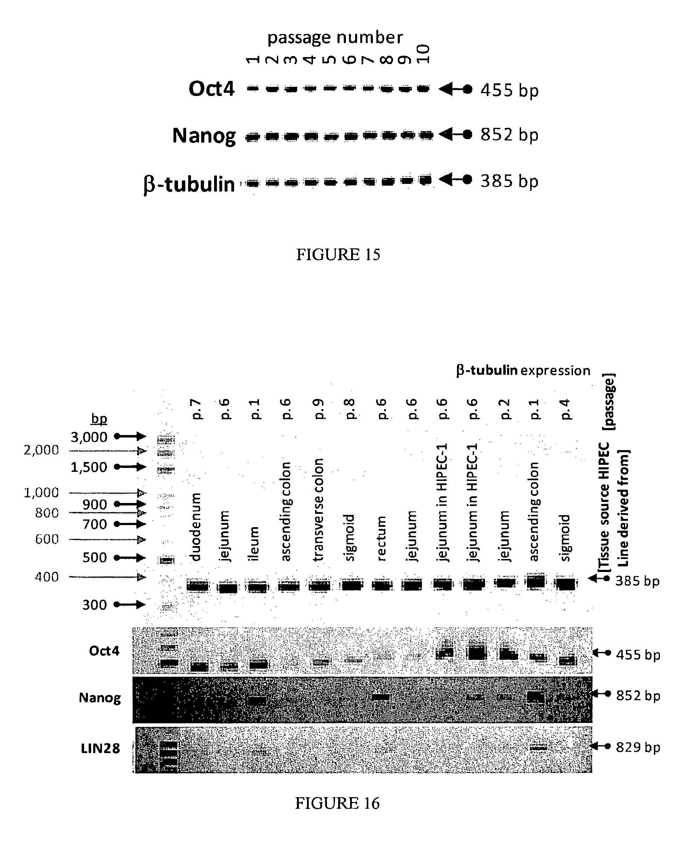

**Figure 16** shows the RT-PCR products from total RNA preparation of cell lines derived from the gastrointestinal tract. Lane: (1) molecular weight marker; (2) duodenum derived stem cell line; (3) jejunum derived stem cell line; (4) ileum derived stem cell line; (5) ascending colon derived stem cell line; (6) transverse colon derived stem cell line; (7) sigmoid derived stem cell line; (8) rectum derived stem cell line; (9) jejunum derived stem cell line in HIPEC-1; (10) jejunum derived stem cell line in HIPEC-1; (11) jejunum derived stem cell line; (12) ascending colon derived stem cell line; (13) sigmoid derived stem cell line.
**Figure 17** shows an agarose gel of the RT-PCR products of a total RNA preparation from cell line A2J1. Lane: (1) molecular weight markers; (2) no template control; (3) KRT18; (4) ITGb1; (5) CHGA; (6) LYZ; (7) ALP1; (8) SI; (9) MUC2; (10) DEFA5; (11) TFF3.
**Figure 18** shows micrographs of which cells immunochemically stained with antibody for CK18, EP4, SC, and MUC2.
**Figure 19** shows electron microscopy images of A2J1 (jejunal) cell line. TJ=tight junction; MEM=membrane (transwell); Mu=mucin; Go=goblet cell appearance; InL=intracellular lumen; C=columnar cells; Emo=elongated monolayer; NC=necrotic cell; BM=basement membrane; and A=apoptotic cell.
**Figure 20** shows electron micrographs of one representative adult human oral mucosal stem cell derived non-transformed primary epithelial cell line grown on transwell membrane (Costar). MV=Microvilli; TJ=intercellular tight junction; L=mucin-containing vesicles.
**Figure 21** shows the RT-PCR products from a total RNA preparation of HIPEC cell lines. Lane: (M) molecular weight; (1) duodenum derived stem cell line; (2) jejunum derived stem cell line; (3) ileum derived stem cell line; (4) ascending colon derived stem cell line; (5) transverse colon derived stem cell line; (6) sigmoid derived stem cell line; and (7) rectum derived stem cell line. SI=sucrase isomaltase. All cell lines expressed the β-tubulin positive control.
**Figure 22** shows immunochemical staining of HIPEC lines using antibodies against vimentin (panel A, red) and cytokeratin-18 (panel B, green).
**Figure 23** shows plots of fluorescence intensity versus cell number of flow cytometric analysis data for epithelial marker cytokeratin-18 (CK; red) and mesenchymal marker vimentin (VIM; blue) expression on HIPECs at various points from passages 1 to 18.
**Figure 24** shows micrographs of a monolayer formed by isolated human gastrointestinal epithelial stem cell derived progenitor cells on a biosimilar matrix. **A**) Light microscopic view of A2J1 cell monolayer (paraformaldehyde-fixed) grown on a biosimilar matrix coated membrane; **B**) immunofluorescence staining (green) of cell:cell junction forming protein E-cadherin and DAPI staining (blue) of the nucleus of the cells in the monolayer.
**Figure 25** shows the percent viability of the cultures as determined by MTT plotted against SN-38 concentration.
**Figure 26** shows a bar graph illustrating IL-8 (µg/ml) production by HIPECs versus medium conditions.

### DETAILED DESCRIPTION

The term "analyze" (or "analysis") as used herein refers to the process whereby a material is separated into constituent parts or elements or essential features. Analyses according to the described invention may be performed by numerous assays including, but not limited to, ELISA, HPLC, PCR, real-time PCR, permeability assays, immunochemistry, flow cytometry, TEER, SDS-PAGE, microscopic analysis, fluorescence microscopy, electron microscopy, NMR, LC-MS, or other analytical or bioanalytical assays known to artisans of skill in the art.

The term "antibody" as used herein refers to both polyclonal and monoclonal antibodies of any species. The ambit of the term encompasses not only intact immunoglobulin molecules, but also fragments and genetically engineered derivatives of immunoglobulin molecules and equivalent antigen binding molecules that retain the desired binding specificity.

The term "AUC" as used herein refers to the area under the plasma concentration-time curve for a single dose of a drug as described in Shargel and Yu, Applied Biopharmaceutics and Pharmacokinetics, 4th Edition, 1999, Appleton & Lange, Stamford, Conn., The AUC is proportional to the amount of drug that reaches the plasma.

The term "bioavailability" as used herein refers to the rate and extent to which an active or therapeutic ingredient of a therapeutic agent or drug is absorbed and becomes available at the site of drug action.

The terms "bio-similar matrix environment" and "BSME" are used interchangeably herein to refer to a growth substrate upon which human gastrointestinal epithelial stem-cell-like progenitor cells may be grown. A segment-specific BSME (herein referred to as "SS-BSME") is formed when each BSME is supplemented with gastrointestinal mucosal tissue derived growth supporting factors (MTD-GSF) appropriate for the isolated viable stem-cell-like progenitor cells of the gastrointestinal mucosal tissue segment of the human gastrointestinal tract that the BSME is to host. Thus, in some embodiments, stomach-BSME is supplemented with growth supporting factors derived from the mucosal tissues of the stomach. In some embodiments, duodenum-BSME is supplemented with growth supporting factors derived from the mucosal tissues of the duodenum. In some embodiments, jejunum-BSME is supplemented with growth supporting factors derived from the mucosal tissues of the jejunum. In some embodiments, ileum-BSME is supplemented with growth supporting factors derived from the mucosal tissues of the ileum. In some embodiments, colon-BSME is supplemented with growth supporting factors derived from the mucosal tissues of the colon. In some embodiments, rectum-BSME is supplemented with growth supporting factors derived from the mucosal tissues of the rectum. Each SS-BSME is adjusted to a pH most appropriate for the gastrointestinal epithelial stem-like epithelial progenitor cell it is to host. Thus, the stomach-BSME is adjusted to about pH 1.0 to about pH 2.0. The duodenum-BSME is adjusted to about pH 4.0 to about pH 5.5. The jejunum-BSME is adjusted to about pH 5.5 to about pH 7.0. The ileum-BSME is adjusted to about pH 7.0 to about pH 7.5. The colon-BSME and rectum-BSME is adjusted to about pH 7.0 to about pH 7.5.

The term "chamber" as used herein refers to culture tubes, Petri dishes, microtiter plates, conical tubes, perfusion chambers, or any type of vessel useful in propagating and/or maintaining cells.

The term "crypt" as used herein refers to a pit-like depression or tubular recess. For example, within the gastrointestinal tract, at the base of the intestinal villi lie crypts where the epithelial cells proliferate.

The term "daughter cell" as used herein refers to one of the resultant cells that is generated when a cell undergoes cell division and divides into two cells. A cell that undergoes cell division and divides into two cells is referred to as a "parent" cell.

The term "differentiation" or "cellular differentiation" as used herein refers to the process by which a less specialized cell becomes a more specialized cell type. In adults, adult stem cells divide and create fully differentiated daughter cells during tissue repair and during normal cell turnover. Cell differentiation causes a cells' size, shape, polarity, metabolic activity, and responsiveness to signals to change dramatically. These changes largely are due to highly controlled modifications in gene expression. Cellular differentiation rarely involves a change in the DNA sequence itself; thus; different cells may have very different physical characteristics despite having the same genome. The term "differentiated" as used herein refers to having a different character or function from the surrounding structures or from the original type. The term "differentiable" as used herein refers to the ability to undergo differentiation or to become differentiated.

The term "disease" or "disorder" as used herein refers to an impairment of health or a condition of abnormal functions. The term "diseased state" as used herein refers to being in a condition of disease or disorder. The term "syndrome" as used herein refers to a pattern of symptoms indicative of some disease or condition. The term "condition" as used herein refers to a variety of health states and is meant to include disorders or disease caused by any underlying mechanism or disorder.

Diseases of the human gastrointestinal tract include, but are not limited to, achalasia, Barrett's oesophagus, colorectal cancer, gastric cancer, oesophageal cancer, coeliac disease, colitis, Crohn's disease, diverticulosis, diverticulitis, gastritis, inflammatory bowel disease, ulcerative colitis, irritable bowel syndrome, microscopic colitis, collagenous colitis, lymphocytic colitis, pancreatitis, reflux oesophagitis, and ulcerative colitis.. Disease states often are quantified in the art using well known scoring systems, such as those elucidated in Goodman & Gilman's The Pharmacological Basis of Therapeutics, 10th Edition, Eds. J. G. Hardman and L. E. Limbird, McGraw-Hill Publishing, New York, NY, 2001.

The term "dissolution" as used herein refers to the ability of a therapeutic agent to pass into a solution in a specific microenvironment determined by the highly interdependent influences of aqueous solubility, ionizability (pKa), and lipophilicity in the gastrointestinal environment. A "solution" generally is considered as a homogenous mixture of two or more substances. It is frequently, although not necessarily, a liquid. In a solution, the molecules of the solute (or dissolved substance) are uniformly distributed among those of the solvent.

The term "drug" as used herein refers to a therapeutic agent or any substance, other than food, used in the prevention, diagnosis, alleviation, treatment, or cure of disease. A drug is: (a) any article recognized in the official United States Pharmacopeia, official Homeopathic Pharmacopeia of the United States, or official National Formulary, or any supplement to any of them; (b) articles intended for use in the diagnosis, cure, mitigation, treatment, or prevention of disease in man or other animals; (c) articles (other than food) intended to affect the structure or any function of the body of man or other animals, and d) articles intended for use as a component of any articles specified in (a), (b) or (c) above.

The term "efficacy" as used herein means a therapeutic agent's capacity to produce a therapeutically desired effect. Generally, a greater level of efficacy will be achieved by increasing the dose and/or frequency of administration of a therapeutic agent given to a population, such that a greater proportion of the population will receive a benefit and/or there will be a greater magnitude of benefit in an individual patient, or cell. If a first therapeutic agent is more potent than a second therapeutic agent, it will reach a greater level of efficacy than the second therapeutic agent using identical amounts of each.

The term human "gastrointestinal epithelial stem cell-like progenitor cell" as used herein refers to a cell having the phenotype cytokeratin(+), β-1-integrin(+), defensin-5(+), trefoil factor-3(+), mucin-2(+), chomogranin-A(+), intestinal alkaline phosphatase(+), lysozyme(+) or at least β-1-integrin⁽⁺⁾ and cytokeratin⁽⁺⁾.

The term "gastrointestinal mucosal tissue segments" as used herein refers to isolated anatomical segments of the human gastrointestinal tract. Gastrointestinal mucosal tissue segments include those prepared from the stomach, the duodenum, the jejunum, the ileum, the ascending colon, the transverse colon, the sigmoid, and the rectum.

The term "HIPEC" as used herein refers to human intestinal primary epithelial cell lines derived from gastrointestinal epithelial stem cell-like progenitor cells according to the disclosed methods. The term "HGISC" as used herein refers to human gastrointestinal stem cells.

The term "human gastrointestinal tract" as used herein refers to the coordinated structure having the function of ingesting and absorbing nutrients and excreting unabsorbed and waste products.

As used herein the term "inflammation" refers to a physiologic response to infection and injury in which cells involved in detoxification and repair are mobilized to the compromised site by inflammatory mediators. The classic signs of inflammation are pain (dolor), heat (calor), redness (rubor), swelling (tumor), and loss of function (functio laesa). Histologically, inflammation involves a complex series of events, including dilatation of arterioles, capillaries, and venules, with increased permeability and blood flow; exudation of fluids, including plasma proteins; and leukocytic migration into the inflammatory focus.

The term "acute inflammation" as used herein, refers to inflammation, usually of sudden onset, characterized by the classical signs, with predominance of the vascular and exudative processes. The term "chronic inflammation" as used herein refers to inflammation of slow progress and marked chiefly by the formation of new connective tissue; it may be a continuation of an acute form or a prolonged low-grade form, and usually causes permanent tissue damage.

Regardless of the initiating agent, the physiologic changes accompanying acute inflammation encompass four main features: (1) vasodilation, which results in a net increase in blood flow, is one of the earliest physical responses to acute tissue injury; (2) in response to inflammatory stimuli, endothelial cells lining the venules contract, widening the intracellular junctions to produce gaps, leading to increased vascular permeability which permits leakage of plasma proteins and blood cells out of blood vessels; (3) inflammation often is characterized by a strong infiltration of leukocytes at the site of inflammation, particularly neutrophils (polymorphonuclear cells). These cells promote tissue damage by releasing toxic substances at the vascular wall or in uninjured tissue; and (4) fever, produced by pyrogens released from leukocytes in response to specific stimuli.

During the inflammatory process, soluble inflammatory mediators of the inflammatory response work together with cellular components in a systemic fashion in the attempt to contain and eliminate the agents causing physical distress. The term "inflammatory mediators" as used herein refers to the molecular mediators of the inflammatory process. These soluble, diffusible molecules act both locally at the site of tissue damage and infection and at more distant sites. Some inflammatory mediators are activated by the inflammatory process, while others are synthesized and/or released from cellular sources in response to acute inflammation or by other soluble inflammatory mediators. Examples of inflammatory mediators of the inflammatory response include, but are not limited to, plasma proteases, complement, kinins, clotting and fibrinolytic proteins, lipid mediators, prostaglandins, leukotrienes, platelet-activating factor (PAF), peptides and amines, including, but not limited to, histamine, serotonin, and neuropeptides, proinflammatory cytokines, including, but not limited to, interleukin-1, interleukin-4, interleukin-6, interleukin-8, tumor necrosis factor (TNF), interferon-gamma, and interleukin 12.

The term "mucosa" as used herein refers to the mucous tissue lining various tubular structures, which comprises an epithelium, a lamina propria, and in the digestive tract, a layer of smooth muscle (muscularis mucomucosa).

The term "mucosal" as used herein means relating to the mucosa or mucous membrane.

The term "native" as used herein refers to the condition of an organ, molecule, compound, protein, or nucleic acid as it would normally occur in nature. For example, a native human gastrointestinal tract refers to a gastrointestinal tract found within a normal human subject.

The term "oral bioavailability" as used herein refers to the fraction of a drug dose given orally that is absorbed into the plasma after a single administration to a subject. A preferred method for determining the oral bioavailability is by dividing the AUC of a drug dose given orally by the AUC of the same drug dose given intravenously to the same patient, and expressing the ratio as a percent. Other methods for calculating oral bioavailability will be familiar to those skilled in the art, and are described in greater detail in Shargel and Yu, Applied Biopharmaceutics and Pharmacokinetics, 4th Edition, 1999, Appleton & Lange, Stamford, Conn.

The term "permeability" as used herein means the property of being permeable.

The term "permeable" as used herein means permitting the passage of substances (e.g., liquids, gases, heat, etc.), as through a membrane or other structure.

The term "progenitor cell" as used herein refers to an immature or undifferentiated cell population. Progenitor cells have a capacity for self-renewal and differentiation, although these properties may be limited. The majority of progenitor cells lie dormant or possess little activity in the tissue in which they reside. They exhibit slow growth and their main role is to replace cells lost by normal attrition. Upon tissue damage or injury, progenitor cells can be activated by growth factors or cytokines, leading to increased cell division important for the repair process.

The term "regional specificity" as used herein refers to the ability of a therapeutic agent to affect a specific identified segment of the human gastrointestinal tract.

The term "specificity" as used herein refers to the ability of a biological molecule to selectively affect a target substance and to not affect other substances commonly recognized by nonselective biological molecules of a similar type; for example, an antibody that binds to an antigen.

The term "stem cell" as used herein refers to undifferentiated cells having high proliferative potential with the ability to self-renew that can generate daughter cells that can undergo terminal differentiation into more than one distinct cell type. A cell that is able to differentiate into many cell types may be referred to as "pluripotent." A cell that is able to differentiate into all cell types may be referred to as "totipotent." Pluripotent stem cells undergo further specialization into multipotent progenitor cells that then give rise to functional cells. Examples of stem and progenitor cells include, but are not limited to,: hematopoietic stem cells (adult stem cells) from the bone marrow that give rise to red blood cells, white blood cells, and platelets; mesenchymal stem cells (adult stem cells) from the bone marrow that give rise to stromal cells, fat cells, and types of bone cells; epithelial stem cells (progenitor cells) that give rise to the various types of skin cells; and muscle satellite cells (progenitor cells) that contribute to differentiated muscle tissue.

The term "therapeutic agent" as used herein refers to a drug, molecule, nucleic acid, protein, composition or other substance that provides a therapeutic effect.

The term "therapeutic component" as used herein refers to a therapeutically effective dosage (i.e., dose and frequency of administration) that eliminates, reduces, or prevents the progression of a particular disease manifestation in a percentage of a population. An example of a commonly used therapeutic component is the ED₅₀ which describes the dose in a particular dosage that is therapeutically effective for a particular disease manifestation in 50% of a population.

The term "therapeutic effect" as used herein refers to a consequence of treatment, the results of which are judged to be desirable and beneficial. For example, a therapeutic effect may include, directly or indirectly, the arrest, reduction, or elimination of a disease manifestation. A therapeutic effect may also include, directly or indirectly, the arrest, reduction or elimination of the progression of a disease manifestation. A therapeutic effect may directly or indirectly kill the diseased cells, arrest the accumulation of diseased cells, or reduce the accumulation of diseased cells in a human subject with a disease such as, but not limited to, achalasia, Barrett's esophagus, colorectal cancer, gastric cancer, esophageal cancer, coeliac disease, colitis, Crohn's disease, diverticulosis, diverticulitis, gastritis, inflammatory bowel disease, ulcerative colitis, irritable bowel syndrome, microscopic colitis, collagenous colitis, lymphocytic colitis, pancreatitis, reflux esophagitis, and ulcerative colitis.

The terms "therapeutically effective amount" and "pharmaceutically effective amount" are used interchangeably to refer to the amount that results in a therapeutic beneficial effect. The term as used herein also refers to the dosage of a therapeutic agent that directly or indirectly reduces or increases the activity of molecules secreted by diseased and/or non-diseased cells participating in a disease manifestation, such that the amount of therapeutic agent arrests, reduces, or eliminates altogether the degree of the disease manifestation. Typically, a therapeutically effective amount will also eliminate, reduce, or prevent the progression of one or more diseases. A skilled artisan recognizes that in many cases a therapeutic agent may not provide a cure, but may provide only a partial benefit. Furthermore, the skilled artisan recognizes that because individual patients and disease states may vary, some patients may receive little, or no benefit at all. A dosage of therapeutic agent that "kills," "arrests," "reduces," or "eliminates" as described above, in at least some patients, is considered therapeutically effective. The term "dosage" as used herein refers to the dose or amount, and frequency of administering of a therapeutic agent in prescribed amounts and frequency. The term "dose" as used herein refers to the amount of therapeutic agent to be taken or applied all at one time or in fractional amounts within a given period.

The term "therapeutic target" as used herein refers to a native protein, molecule, compound, nucleic acid, organ, gland, ligand, receptor, organelle, or cell whose activity is modified by a drug resulting in a desirable therapeutic effect.

The term "trans-epithelial electrical resistance" ("TEER") as used herein refers to a functional assay that detects nanoscale alterations of an epithelial test barrier.

The term "transport" as used herein refers to the movement or transference of biochemical substances in biologic systems. "Active transport" refers to the passage of ions or molecules across a cell membrane, not by passive diffusion, but by an energy-consuming process at the expense of catabolic processes proceeding within the cell; in active transport, movement takes place against an electrochemical gradient. "Facilitated (or passive) transport" refers to the protein-mediated transport of a compound across a biomembrane that is not ion-driven and is saturable. "Paracellular transport" refers to solvent movement across an epithelial cell layer through the tight junctions between cells. "Transcellular transport" refers to transport of macromolecules across a cell, including transport through channels, pumps, and transporters, as well as transcytosis (endocytosis of macromolecule at one side of a monolayer and exocytosis at the other side).

As used herein the term "treating" includes abrogating, substantially inhibiting, slowing or reversing the progression of a condition, substantially ameliorating clinical or aesthetical symptoms of a condition, substantially preventing the appearance of clinical or aesthetical symptoms of a condition, and protecting from harmful or annoying stimuli.

The term "toxicity" as used herein refers to any undesired harmful effect of a therapeutic agent. A therapeutic agent is said to be toxic or have toxicity if it causes a toxicity manifestation in a percentage of a population.

The term "villi" as used herein refers to projections from the surface, especially of a mucous membrane. In nature, intestinal villi are projections, about 0.5 mm to about I .5 mm in length, of the mucous membrane of the small intestine. They are leaf-shaped in the duodenum and become shorter, more finger-shaped, and sparser in the ileum.

In one aspect, the described invention provides methods for the isolation of gastrointestinal epithelial stem cell-like progenitor cells located in the gastrointestinal mucosal tissues, growth and differentiation of such cells to specific types of epithelial cell lineages (e.g., columnar epithelium, goblet cell, Paneth cell and/or enteroendocrine chromaffin cells), maintenance of these cells in a non-transformed state, and use of these cells as a model system in drug discovery.

Human gastrointestinal epithelial stem-cell-like progenitor cells according to the described invention are derived from monolayers of epithelial cells and can be grown in culture for long periods of time without transformation. Upon establishment of a monolayer, cells are polarized (apical or basolateral) and joined by tight junctions mimicking the actual physiological scenario. The cells can be grown in large quantities and stably stored in liquid nitrogen for extended periods. The gene expression profile of these cells is the same as that observed in the human gastrointestinal epithelium.

In another aspect, the described invention provides a system to reproducibly characterize the bioavailability of a therapeutic agent comprising epithelial monolayers derived from each segment of the gastrointestinal tract, which allows for physiologically relevant regional and/or segmental comparison. It also provides methods for region specific evaluation of absorption and transport of any therapeutic agent. The system provides an accurate representation of the physiological environment of at least one gastrointestinal segment and allows for assessment of specific changes within the gastrointestinal epithelial function early in the drug development timeline. The method comprises several steps.

First, viable human gastrointestinal epithelial stem-cell-like progenitor cells are isolated from at least one segment of the gastrointestinal mucosal tissue of a human subject, wherein the segment of gastrointestinal mucosal tissue comprises a plurality of gastrointestinal mucosal tissue segments. According to one embodiment, gastrointestinal mucosal tissue segments are prepared from the stomach, duodenum, jejunum, ileum, ascending colon, transverse colon, sigmoid, and/or rectum.

Second, a segment-specific bio-similar matrix environment (SS-BSME) is prepared from each segment of the gastrointestinal mucosal tissue. According to one embodiment, an SS-BSME prepared from stomach tissue segments is a stomach-specific bio-similar-matrix-environment. According to another embodiment, an SS-BSME prepared from duodenum tissue segments is a duodenum-specific bio-similar-matrix-environment. According to another embodiment, an SS-BSME prepared from jejunum tissue segments is a jejunum-specific bio-similar-matrix-environment. According to another embodiment, an SS-BSME prepared from ileum tissue segments is an ileum-bio-similar-matrix-environment. According to another embodiment, an SS-BSME prepared from colon tissue segments is a colon-specific bio-similar-matrix-environment. According to another embodiment, an SS-BSME prepared from rectum tissue segments is a rectum-specific bio-similar-matrix-environment.

Third, the viable gastrointestinal epithelial stem-cell-like progenitor crypt cells are seeded onto an individual SS-BSME derived from the viable stem-cell-like progenitor crypt cells corresponding to a gastrointestinal mucosal tissue segment.

Fourth, the BSME are incubated to allow for the formation of a monolayer of gastrointestinal epithelial stem-cell-like-progenitor cells.

Fifth, the gastrointestinal epithelial stem-cell-like-progenitor cells of each BSME are treated with a therapeutic agent.

Sixth, the gastrointestinal epithelial stem-cell-like-progenitor cells of each BSME are analyzed to characterize the bioavailability of the therapeutic agent.

In another aspect, the described invention provides methods to identify therapeutic targets in cells representative of a segment of gastrointestinal mucosa.

According to some embodiments, the individual BSMEs are dispensed into chambers. In some embodiments, the chambers may be culture tubes, Petri dishes, conical tubes, or any type of chamber, container, or vessel that allows propagation and maintenance of cells *in vitro.* In some embodiments, the chambers may be arranged in any desired order, including, but not limited to, sequentially, randomly, or individually.

According to one embodiment, individual BSMEs are sequentially arranged in the order of a stomach-BSME, a duodenum-BSME, ajejunum-BSME, an ileum-BSME, an ascending colon-BSME, a transverse colon-BSME, a sigmoid-BSME, and a rectum-BSME to mimic the structure of the human gastrointestinal tract. According to other embodiments, the individual BSMEs are formed and utilized separately and/or in discrete subunits where the composition of each subunit is determined by the user.

According to another embodiment, BSMEs are connected to each other via tubes, hoses, valves, check-valves, Y-connectors, connectors or other means suitable for the passage of fluids, vapors, or solids. For example, BSMEs representing each of the segments of the gastrointestinal tract may be connected to each other serially to form an *in vitro* model of the human gastrointestinal tract. Alternately, selected BSMEs may be connected to each other such that an *in vitro* model of a portion of the human gastrointestinal tract is formed.

According to another embodiment, each BSME may be polarized, so that it has apical and basolateral surfaces.

According to another embodiment, the electrical resistance of the gastrointestinal epithelial stem-cell-like-progenitor cells is comparable to that of the gastrointestinal epithelial stem-cell-like progenitor crypt cells of the native human gastrointestinal tract. In some such embodiments, TEER values of bio-similar-matrix environments (BSMEs) with gastrointestinal epithelial stem-cell-like progenitor crypt cells is about 25-70 ohm/cm². See infra, **Figure 11**.

According to another embodiment, the gastrointestinal epithelial stem-cell-like progenitor cells derived from each BSME are genetically modified to a state such that, upon reintroduction into a human patient, the cells are useful for the treatment of gastrointestinal diseases.

In another aspect, the gastrointestinal epithelial stem-cell-like progenitor cells derived from each BSME may be used to assess cellular toxicity of the therapeutic agent. In some embodiments, for example, the gastrointestinal epithelial stem cell-like progenitor cells of the described invention may be used to determine variations in DNA and/or RNA characteristics as related to a response of such cells to a therapeutic agent. As used herein, the term "genomic biomarker" refers to a measurable DNA or RNA characteristic that is an indicator of normal biologic or pathogenic processes or a response to a therapeutic or other interventions. As used herein, the term "pharmacogenomics" refers to the study of variations of DNA and RNA characteristics as related to a drug response, and the term "pharmacogenetics" refers to the study of variations in DNA sequence as related to a drug response. DNA characteristics include, but are not limited to, single nucleotide polymorphisms (SNPs), variability of short sequence repeats, haplotypes, DNA modifications (e.g. methylation), deletions or insertions of (a) single nucleotide(s), copy number variations, and cytogenetic rearrangements (e.g., translocations, duplications, deletions or inversions). RNA characteristics include, but are not limited to, RNA sequences, RNA expression levels, RNA processing (e.g., splicing and editing), and microRNA levels.

In another aspect, the described invention provides a method of making an implantable mucosal scaffold comprising the steps of combining at least two BSMEs such that the stromal tissue underneath is combined with the epithelial monolayers of the gastrointestinal epithelial stem-cell-like progenitor cells derived from each BSME to form a three-dimensional structure of the intestinal mucosa. In one embodiment, the number of different gastrointestinal mucosal tissue segments represented by a corresponding BSME is from about 1 through about 8.

In another aspect, each gastrointestinal epithelial stem-cell-like-progenitor cells-BSME pairing has region specific functional properties. In one embodiment, the gastrointestinal epithelial stem-cell-like-progenitor cells derived from the stomach-BSME have region specific functional properties characteristic of the stomach epithelium. In another embodiment, the gastrointestinal epithelial stem-cell-like-progenitor cells derived from the duodenum-BSME have region specific functional properties characteristic of the duodenum epithelium. In another embodiment, the gastrointestinal epithelial stem-cell-like-progenitor cells derived from the jejunum-BSME have region specific functional properties characteristic of the jejunum epithelium. In another embodiment, the gastrointestinal epithelial stem-cell-like progenitor cells derived from the ileum-BSME have region specific functional properties characteristic of the ileum epithelium. In another embodiment, the gastrointestinal epithelial stem-cell-like-progenitor cells derived from the ascending colon-BSME have region specific functional properties characteristic of the ascending colon epithelium. In another embodiment, the gastrointestinal epithelial stem-cell-like-progenitor cells derived from the transverse colon-BSME have region specific functional properties characteristic of the transverse colon epithelium. In another embodiment, the gastrointestinal epithelial stem-cell-like-progenitor cells derived from the sigmoid-BSME have region specific functional properties characteristic of the sigmoid epithelium. In another embodiment, the gastrointestinal epithelial stem-cell-like-progenitor cells derived from the rectum-BSME have region specific functional properties characteristic of the rectum epithelium.

In another embodiment, the permeability of the gastrointestinal epithelial stem-cell-like-progenitor cells-BSME is assayed at about pH 7.4 or with a pH gradient.

In another embodiment, the gastrointestinal epithelial stem-cell-like progenitor cells isolated from gastrointestinal mucosal tissue segments may differentiate into a mature adult cell phenotype. In one such embodiment, the mature cell phenotype is a columnar epithelial cell. In another such embodiment, the mature cell phenotype is a Paneth cell. In another such embodiment, the mature cell phenotype is a goblet cell. In another such embodiment, the mature cell phenotype is an enteroendocrine chromaffin cell. In another such embodiment, the cell is a mesenchymal cell. In another such embodiment, the mature cell phenotype is a neuronal cell type.

In another aspect, the bioavailability of a therapeutic agent is determined by measuring the permeability profile of the gastrointestinal epithelial stem-cell-like-progenitor cells of each BSME. In some such embodiments, the gastrointestinal epithelial stem-cell-like-progenitor cells of each BSME are assayed for permeability of a therapeutic agent of high, medium and/or low solubility. In some such embodiments, the gastrointestinal epithelial stem-cell-like-progenitor cells of each BSME are assayed for permeability of a therapeutic agent in a nonionized and/or ionized form. In some such embodiments, the gastrointestinal epithelial stem-cell-like-progenitor cells of each BSME are assayed for permeability of a therapeutic agent in a lipophilic and/or nonlipophilic form. In some such embodiments, the gastrointestinal epithelial stem-cell-like-progenitor cells of each BSME are assayed for permeability of a therapeutic agent resistant and/or nonresistant to gastric juices. In some such embodiments, the gastrointestinal epithelial stem-cell-like-progenitor cells of each BSME are assayed for permeability of a therapeutic agent with and/or without components of protective coatings. In some such embodiments, the gastrointestinal epithelial stem-cell-like-progenitor cells of each BSME are assayed for permeability of a therapeutic agent designed to be controlled-release. In some such embodiments, the gastrointestinal epithelial stem-cell-like-progenitor cells of each BSME are assayed for permeability of a therapeutic agent designed to be extended-release. In some such embodiments, the gastrointestinal epithelial stem-cell-like-progenitor cells of each BSME are assayed for permeability of a therapeutic agent designed to be sustained-release. In some such embodiments, the gastrointestinal epithelial stem-cell-like-progenitor cells of each BSME are assayed for permeability of a therapeutic agent designed to be a prolonged-action pharmaceutical preparation that is designed to produce slow, uniform absorption of the therapeutic agent for 8 hours or longer.

In another aspect of the described invention, a method to identify an effective therapeutic agent to treat a specific disease of the gastrointestinal tract comprises the following steps. First, viable gastrointestinal epithelial stem-cell-like progenitor cells are isolated from the gastrointestinal mucosal tissues of a human subject, wherein the gastrointestinal mucosal tissue is comprised of gastrointestinal mucosal tissue segments, wherein at least one of the gastrointestinal mucosal tissue segments is in a diseased state. Second, SS-BSMEs are prepared from each gastrointestinal mucosal tissue segment. Third, the viable gastrointestinal epithelial stem-cell-like-progenitor cells are seeded onto the SS-BSME derived from the gastrointestinal epithelial stem-cell-like-progenitor cells. Fourth, the BSMEs are incubated to allow for the formation of a monolayer of gastrointestinal epithelial stem-cell-like-progenitor cells. Fifth, the gastrointestinal epithelial stem-cell-like-progenitor cells of each BSME are exposed to a candidate therapeutic agent. Sixth, the gastrointestinal epithelial stem-cell-like-progenitor cells of each BSME are analyzed to identify a desired therapeutic effect.

In another aspect, the described invention provides a method to ascertain regional specificity of a therapeutic agent useful in treating disorders of the gastrointestinal tract. The method comprises several steps. First, viable gastrointestinal epithelial stem-cell-like-progenitor cells are isolated from the gastrointestinal mucosal tissues of a human, wherein the gastrointestinal mucosal tissue is comprised of gastrointestinal mucosal tissue segments. Second, an individual BSME is formed from each gastrointestinal mucosal tissue segment. Third, the viable gastrointestinal epithelial stem-cell-like-progenitor cells are seeded onto an individual BSME derived from the viable gastrointestinal epithelial stem-cell-like-progenitor cells. Fourth, the BSMEs are incubated to allow formation of a monolayer of the gastrointestinal epithelial stem-cell-like-progenitor cells. Fifth, each BSME is treated with a therapeutic agent. Sixth, the gastrointestinal epithelial stem-cell-like-progenitor cells of each BSME are analyzed to determine regional specificity of the therapeutic agent.

In some embodiments, the gastrointestinal epithelial stem-cell-like-progenitor cells demonstrate regional specificity characteristic of areas of the small intestine. In some embodiments, the gastrointestinal epithelial stem-cell-like-progenitor cells demonstrate regional specificity characteristic of at least areas of the large intestine. In some embodiments, the gastrointestinal epithelial stem-cell-like-progenitor cells demonstrate regional specificity characteristic of at least areas of the stomach. In some embodiments, the gastrointestinal epithelial stem-cell-like-progenitor cells demonstrate regional specificity characteristic at least of areas of the duodenum. In some embodiments, the gastrointestinal epithelial stem-cell-like-progenitor cells demonstrate regional specificity characteristic at least of areas of the jejunum. In some embodiments, the gastrointestinal epithelial stem-cell-like-progenitor cells demonstrate regional specificity characteristic at least of areas of the ileum. In some embodiments, the gastrointestinal epithelial stem-cell-like-progenitor cells demonstrate regional specificity characteristic at least of areas of the ascending colon. In some embodiments, the gastrointestinal epithelial stem-cell-like-progenitor cells demonstrate regional specificity characteristic at least of areas of the transverse colon. In some embodiments, the gastrointestinal epithelial stem-cell-like-progenitor cells demonstrate regional specificity characteristic at least of areas of the sigmoid colon. In some embodiments, the gastrointestinal epithelial stem-cell-like-progenitor cells demonstrate regional specificity characteristic at least of areas of the rectum.

Gastrointestinal epithelial stem-cell-like progenitor cells may be propagated *in vitro* by providing a suitable surface and a suitable mixture of soluble factors. An autologous composition of growth factors derived from corresponding mucosal tissue, along with mucosal tissue obtained gastrointestinal epithelial stem-cell-like progenitor cells, are required for viable growth and differentiation of the stem-cell-like progenitor crypt cells.

It has been found that gastrointestinal epithelial stem-cell-like progenitor cells grown in bio-similar-matrix-environments grow to form viable monolayers while retaining normal cellular characteristics.

General methods in molecular genetics and genetic engineering useful in the described invention are described in the current editions of Molecular Cloning: A Laboratory Manual, (Sambrook et al., Cold Spring Harbor); Gene Transfer Vectors for Mammalian Cells (Miller & Calos eds.); and Current Protocols in Molecular Biology (F. M. Ausubel et al. eds., Wiley & Sons). Cell biology, protein chemistry, and antibody techniques can be found in Current Protocols in Protein Science (J. E. Colligan et al. eds., Wiley & Sons); Current Protocols in Cell Biology (J. S. Bonifacino et al., Wiley & Sons) and Current Protocols in Immunology (J. E. Colligan et al. eds., Wiley & Sons.). Reagents, cloning vectors, and kits for genetic manipulation are available from commercial vendors, such as BioRad, Stratagene, Invitrogen, ClonTech, and Sigma-Aldrich Co.

Cell culture methods useful in the described invention are described generally in the current edition of Culture of Animal Cells: A Manual of Basic Technique (R. I. Freshney ed., Wiley & Sons); General Techniques of Cell Culture (M. A. Harrison & I. F. Rae, Cambridge Univ. Press), and Embryonic Stem Cells: Methods and Protocols (K. Turksen ed., Humana Press). Other relevant texts are Creating a High Performance Culture (Aroselli, Hu. Res. Dev. Pr. 1996) and Limits to Growth (D. H. Meadows et al., Universe Publ. 1974). Tissue culture supplies and reagents are available from commercial vendors such as GibcoBRL, Nalgene-Nunc International, Sigma Chemical Co., and ICN Biomedicals.

Unless defined otherwise, all technical and scientific terms used herein have the same meaning as commonly understood by one of ordinary skill in the art to which this invention belongs.

It must be noted that as used herein and in the appended claims, the singular forms "a", "and" and "the" include plural references unless the context clearly dictates otherwise. All technical and scientific terms used herein have the same meaning.

Publications discussed herein are provided solely for their disclosure prior to the filing date of the described application. Nothing herein is to be construed as an admission that the present invention is not entitled to antedate such publication by virtue of prior invention. Further, the dates of publication provided may be different from the actual publication dates which may need to be independently confirmed.

### EXAMPLES

The following examples are put forth so as to provide those of ordinary skill in the art with a complete disclosure and description of how to make and use the described invention, and are not intended to limit the scope of what the inventors regard as their invention nor are they intended to limit the scope of what the inventors regard as their invention nor are they intended to represent that the experiments below are all or the only experiments performed. Efforts have been made to ensure accuracy with respect to numbers used (e.g., amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1. Isolation of Human Gastrointestinal Epithelial Segment-Specific Stem-Cell-Like Progenitor Cells

### Example 1(a) Tissue Isolation

Tissue from resected specimens or biopsies from various segments of the gastrointestinal tract (from the mouth to the rectum) were transported from an operating room to the laboratory either in University of Wisconsin medium (UW), normal saline or in RPMI medium supplemented with antibiotics (penicillin (100 Units/ml and streptomycin (100 µg/ml) (Gibco)) in wet ice within 24 hours from the removal of the specimen. The specimens were rinsed vigorously in sterile phosphate buffered saline (1X), pH 7.4 (PBS) (Invitrogen, Carlsbad, CA; Catalog No. 10010-023) to remove loosely adherent material, gently rubbed with sterile gauze, and washed at least ten times in PBS supplemented with antibiotic (1% penicillin/streptomycin) (ICN Biomedical, Costa Mesa, CA) and antimycotic (1% Fungizone) ("supplemented PBS").

The mucosa was stripped off by careful dissection from the underlying submucosa and minced into small pieces (see Panja, A. Lab. Invest. 2000. 80:9; 1473-1475). Tissue pieces were treated with 1 mM dithiothreitol for 15 minutes (Sigma Chemical Co., St. Louis, MO) in RPMI 1460 (Gibco).

### Example 1(b) Generation of Mucosal Tissue Derived Growth Supporting Factors (MTD-GSF)

A portion of the mucosal tissue pieces (approximately 5 grams or one third of the specimen) was cultured overnight in serum free minimum essential medium (MEM) (250 mg/ml) to generate culture supernatants containing autologous growth factor(s)/cytokines that would mimic the cytokine milieu of the mucosal site. The collected supernatant was diluted (1:100) in F-12 medium (Gibco/Invitrogen, Carlsbad, CA) and supplemented with hydrocortisone (15 µM), transferin (10 µg/ml), insulin (0.2 units/ml), epidermal growth factor (250 nM), and retinoic acid (5 µg/ml) (Sigma-Aldrich, St. Louis, MO) to form Human Gastrointestinal Stem Cell medium, referred to hereafter as "HGISC medium."

### Example 1(c) Cell Isolation

The remaining tissue pieces were processed for isolation of surface and crypt epithelial cells by using sequential protease (dispase) treatments.

Mucosal tissue segments were treated with dispase solution (0.5 mg/ml in RPMI 1406 (Invitrogen, Carlsbad, CA)) six times for 5 minutes, 15 minutes, 20 minutes, 20 minutes; 30 minutes, and 30 minutes, respectively, each in an orbital shaker at 37°C. At each interval, the cell suspensions were collected and the solution evaluated for the presence of progenitor stem-cell-like crypt epithelial cells that were liberated from the mucosal pieces by the dispase treatments. Histological examination of the sample tissue pieces after each dispase treatment was performed. Hematoxylin and eosin (Sigma Aldrich, St. Louis, MO) staining of the histologic segments demonstrated that the surface epithelium was completely removed by the first two dispase treatments with maintenance of the crypt epithelium. After the fifth treatment, examination of the histologic segments revealed the absence of crypt epithelium. Subsequently, the intestinal epithelial cells isolated in the first three dispase treatments were considered as enriched surface epithelial cells and the cells from the latter fractions (the fourth) were considered crypt epithelial cells. The cells obtained in the third fraction may contain a mixed population of cells.

### Example 2. Formation of Bio-Similar-Matrix-Environments (BSMEs) on Plastic Surfaces

The de-epithelialized mucosal tissues remaining after the dispase treatment of Example 1 were collected and homogenized in PBS (1 ml/1 mg tissue). A cocktail of protease inhibitors, which included PMSF (1 mM), Aprotinin (0.15 units/ml), Lenpeptin (5 µg/ml), Pepstatin (1 µg/ml) and fluoride (1 mM), was added to the homogenate (0.5 ml of protease inhibitor cocktail for 20 grams of tissue lysate) to prevent degradation of matrix protein substances that may provide anchorage and/or support for epithelial growth. The protein concentration of this mucosal tissue homogenate was determined by Bradford protein assay (Sigma-Aldrich, St. Louis, MO) then was diluted into 1 mg/ml in RPMI medium (Invitrogen, Carlsbad, CA) and used to coat the surfaces of plastic Petri dishes for 30 minutes to thereby form a bio-similar-matrix-environment on the plastic surface. In some cases, human collagen type IV (10 µg/ml) (Sigma Aldrich, St. Louis, MO) was used as a supplement. The resulting BSME facilitated the attachment and growth of the isolated gastrointestinal epithelial stem-cell-like progenitor cells.

Six different growth media representative of each segment of the human gastrointestinal tract ("segment-specific-BSME") were prepared, i.e., stomach-BSME; duodenum-BSME; jejunum-BSME; ileum-BSME; colon-BSME; and rectum-BSME. Each segment-specific-BSME (SS-BSME) was supplemented with **m**ucosal **t**issue **d**erived **g**rowth supporting **f**actors (MTD-GSF) appropriate for each segment of the human gastrointestinal tract (supplemented BSME). Thus, stomach-BSME was supplemented with growth supporting factors derived from the mucosal tissues of the stomach; duodenum-BSME was supplemented with growth supporting factors derived from the mucosal tissues of the duodenum; jejunum-BSME was supplemented with growth supporting factors derived from the mucosal tissues of the jejunum; ileum-BSME was supplemented with growth supporting factors derived from the mucosal tissues of the ileum; colon-BSME was supplemented with growth supporting factors derived from the mucosal tissues of the colon; and rectum-BSME was supplemented with growth supporting factors derived from the mucosal tissues of the rectum.

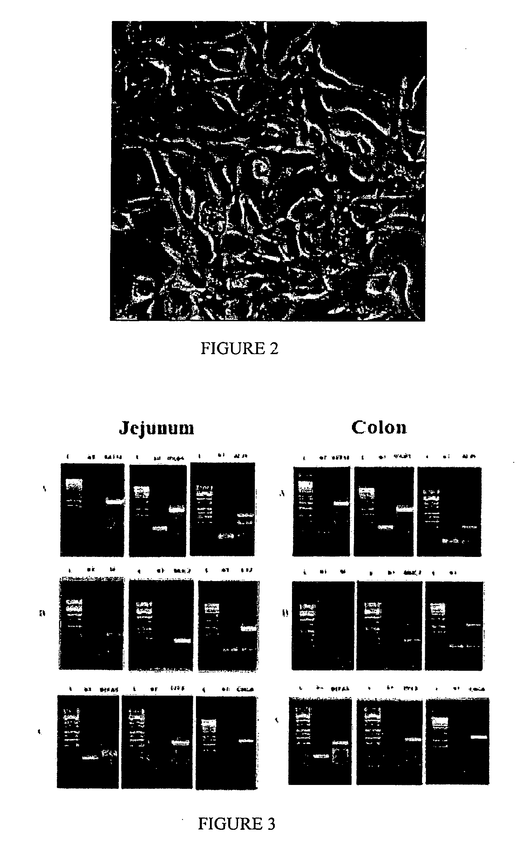

In some embodiments, the supplemented BSME was arranged sequentially in the order: (1) stomach-BSME, (2) duodenum-BSME, (3) jejunum-BSME, (4) ileum-BSME, (5) colon-BSME, and (6) rectum-BSME to mimic the anatomical order of the human gastrointestinal tract. Alternately, it may be arranged in any order the user requires, including non-sequential and vertically (i.e., to form a 3-dimensional representative model of the gastrointestinal tract, or part(s) thereof). **Figure 2** shows a light photomicrograph (20X) of one embodiment of a cell monolayer of an intestinal (jejunal) epithelial stem cell-like progenitor cell population grown on a mucosal derived matrix coated plastic surface in culture. The supplemented BSME medium facilitated the attachment and growth of isolated gastrointestinal epithelial stem-cell-like progenitor cells.

### Example 3. Analysis of Intestinal Epithelial Lineage Specific Molecular Marker Expression

Primary cell culture populations derived from the mucosal tissues of small and large intestinal segments were analyzed by PCR for the mRNA expression of the molecular markers cytokeratin - 18 (CK18) (marker for epithelial lineage), sucrase isomaltase (SI) (small intestinal epithelial enzyme), intestinal alkaline phostase ALPI (marker for columnar epithelial cells), trefoil factor - 3 (TFF3) and mucin - 2 (MUC2) (markers for goblet lineage), defensin (DEF-5) and lysozyme (LYZ) (markers for paneth cells), chomogranin-A (CHGA) (marker for enteroendocrine cells) and β-1 integrin (indicator for stem cell characteristic).

Reverse transcription polymerase chain reaction (RT-PCR) was used to amplify the molecular markers. The RT-PCR process involves four steps: (i) RNA purification; (ii) reverse transcription of the RNA to its DNA complement ("complementary DNA" or "cDNA"); (iii) PCR amplification of the cDNA; and (iv) analysis of the RT-PCR products. An oligo(dT)₂₀ primer was utilized to prime the RT reaction.

### Example 3.1. Purification of Total RNA

Total RNA was purified either from (i) tissue samples/biopsies or (ii) adherent cells growing in tissue culture flasks.

Tissue samples were placed in RNA Later (Invitrogen, Carlsbad, CA) solution and stored at -20°C until processed. Approximately 1 ml of RiboZol RNA Extraction Reagent (Amresco, Solon, OH) was added per 50-100 mg of tissue sample and the sample homogenized using a glass-Teflon probe.

Cells grown in culture were lysed directly in the culture dish by the addition of 1 ml of RiboZol per 10 cm² of dish surface area and repetitive pipetting.

After homogenization, the samples were incubated for 5 minutes at 15-30 °C to allow the dissociation of nucleoprotein complexes, then chloroform was added (0.2 ml chloroform per 1 ml of the RiboZol utilized above to homogenize the cells or tissue samples). Samples were shaken vigorously by hand for 15 seconds, incubated at 15-30 °C for 3 minutes, then centrifuged 12,000 x g for 15 minutes at 2-8 °C. The upper aqueous phase was removed to a fresh tube and isopropanol added (0.5 ml of isopropanol per 1 ml of RiboZol utilized in the initial homogenization). The sample was vortexed for 1 minute, incubated for 10 minutes at 15-30 °C, then centrifuged at 12,000 x g for 10 minutes at 2-8 °C. The supernatant was removed and the RNA pellet washed with 75% ethanol (1 ml of ethanol per 1 ml of RiboZol utilized). The RNA pellet was allowed to air dry and then resuspended in DEPC treated distilled filtered H₂O.

The total RNA purified was measured utilizing a spectrophotometer and absorbance at A₂₆₀. Expected yields of total RNA varied upon tissue and cell type, but averaged between 1-10 µg per mg of starting tissue or 1 x 10⁶ cultured cells.

### Example 3.2. Reverse Transcription Polymerase Chain Reaction (RT-PCR)

### Example 3.2.1. Reverse Transcription (RT)

The RT and PCR reactions and incubations were performed in a thermal cycler with a heated lid (Techne PROGENE®). The reaction mix for the first strand synthesis (RT reaction) included: (a) total RNA template (2.0 µg); (b) 25 µM oligo(dT)₂₀ primer (4 µl); (c) 10 mM dNTP (6.6 µl), and (d) DEPC H₂O to bring the final reaction mix volume to 26.7 µl.

The reaction mix was incubated at 70°C for 5 minutes, then quickly cooled to 4°C to allow the oligo(dT) primer to anneal to the RNA template. A RT cocktail (23.3 µl) then was added to the reaction mix. The RT cocktail included: (a) ImProm-II 5X Reaction Buffer (10 µl) (Promega); (b) Recombinant RNasin Ribonuclease Inhibitor (20-40 U/µl) (2 µl) (Promega); (c) 25 mM MgCl₂ (8 µl) (Promega); and (d) ImProm-II Reverse Transcriptase (3.3 µl) (Promega).

The RT reaction mix then was heated to (i) 25 °C for 5 minutes, (ii) 50 °C for 60 minutes, (iii) 70 °C for 15 minutes, and (iv) cooled to 4 °C. Ribonuclease H (1 µl) (0.5-2.0 U/µl) (Promega) then was added to the RT reaction mix and the RT reaction mix further heated at 37 °C for 30 minutes, then cooled to 4 °C.

The RT reaction product (cDNA) was used as template in the subsequent PCR reactions.

### Example 3.2.2. Polymerase Chain Reaction (PCR)

After completion of the reverse transcription reaction, the cDNA was used as template in standard PCR. Each PCR reaction mix (50 µl) included: (a) RT reaction product (4 µl); (b) 5X Colorless GoTaq Flexi Buffer (10µl) (Promega); (c) 25 mM MgCl₂ (5 µl) (Promega); (d)10 mM dNTP (1 µl); (e) sense/forward target gene specific oligonucleotide primer (3 µl of 10 µM stock); (f) antisense/reverse oligonucleotide primer (3 µl of 10 µM stock); (g) GoTaq Hot Start Polymerase (0.25 µl) (5 U/µl) (Promega), and (h) DEPC H₂O (23.75 µl).

PCR Master mixes (concentrated reaction mixes) containing all components except the cDNA template were made in advance. Then 46.0 µl aliquots of the pre-mixed reagents were added to 4.0 µl of RT product. The PCR reaction was incubated for 2 minutes at 94 °C before proceeding onto 30-45 cycles (dependent upon gene target) of 94 °C for 40 seconds (dennaturation step), 56 °C for 30 seconds (annealing step), and 73 °C for 30-60 seconds (extension step) depending upon the predicted amplification product size (see **Example 10.1** for thermocycling protocols and primers). The reaction was then cooled to 4 °C and kept at -20 °C until analysis.

Master mixes of reagents were utilized in order to minimize variability. Samples for RT-PCR were prepared together, total RNA concentration determined by A₂₆₀, oligo (dT)₂₀ utilized to prime all RT reactions, and the samples equilibrated not just for total RNA content, but also normalized based upon amplification of two control primers sets which amplify beta tubulin and GAPDH. Furthermore, the use of the Low DNA Mass Ladder (Invitrogen) provides a consistent normalization reference from gel to gel via application of its mass characteristic.

These conditions, concentrations, and reagents utilized for the RT-PCR reaction allow for an RT-PCR product assayed in the log phase of amplification (pre-plateau) which was readily visible, making the visualization, measurement, and quantitation of samples both reproducible and consistent with minimal variability. Values are normalized based upon the control RT-PCR amplification of each sample utilizing RT-PCR results of both β-tubulin and GAPDH for each individual sample. Intensity values differences in samples which are greater than 20% of the mean intensity value were considered significant.

**Figure 3** shows RT-PCR products run in a 2% agarose gel (GenePure LE Agarose; ISC BioExpress; catalog number: E-3120-125) in IXTBE buffer (TBE 10X; Amresco; catalog number: 0658-4L) on a Gibco BRL Horizon 58 gel electrophoresis apparatus at 90 Volts utilizing an E-C Apparatus Corporation's EC105 power supply. The agarose gel then was imaged utilizing a BioRad VersaDoc Model 3000 imaging system and quantitated utilizing Quantity One software (BioRad). The agarose gels were not stained with ethidium bromide, instead 10 ul of RT-PCR product was mixed with 2 ul of EZ-Vision One 6X loading dye (Amresco; catalog number: N472-Q-0.5ML) prior to loading onto the agarose gel. A 100 bp DNA MW marker (VWR; catalog number: 95039-808) also was utilized to provide both accurate and consistent MW size and mass measurements.

One embodiment of a primary cell culture derived from isolated gastrointestinal epithelial stem-cell-like progenitor cells from small and large intestinal segments was analyzed by PCR for the presence or absence of various intestinal lineage specific marker expression. As shown in **Figure 3**, PCR analysis confirmed the presence of lineage markers for all four types (columnar, goblet, enteroendocrine, paneth) of epithelial lineages. It also confirmed the presence of a pan epithelial marker (CK18), and at least one known marker (β-1-integrin) for stem cell.

### Example 4. Growth of Isolated Crypt/Progenitor Epithelial Monolayers

The isolated gastrointestinal epithelial stem-cell-like epithelial progenitor cells were prepared as described in Example 1. The cells were cultured for 24 hours in F-12 medium (Mediatech, Inc., Manassas, VA) (1 x 10⁶ cells/ml) supplemented with MTD-GSF (10%) on the appropriate BSME.

Each of the isolated gastrointestinal epithelial stem-cell-like progenitor cell preparation was seeded (between 10 x 10⁴ cells/ml/cm² and 20 x 10⁴ cells/ml/cm²) onto the appropriate media. Thus, the gastrointestinal epithelial stem-cell-like progenitor cells isolated from the stomach were seeded onto the stomach-BSME; the gastrointestinal epithelial stem-cell-like progenitor cells isolated from the duodenum were seeded onto duodenum-BSME; the gastrointestinal epithelial stem-cell-like progenitor cells isolated from the jejunum were seeded onto jejunum-BSME; the gastrointestinal epithelial stem-cell-like progenitor cells isolated from the ileum were seeded onto ileum-BSME; the gastrointestinal epithelial stem-cell-like progenitor cells isolated from the colon were seeded onto colon-BSME; and the gastrointestinal epithelial stem-cell-like progenitor cells isolated from the rectum were seeded onto rectum-BSME.

### Example 5. Differentiation of Stem-Cell-Like Progenitor Cells to a Substantially Pure Population of Epithelial Cells

Cells of epithelial origin contain cytoskeletal keratin. While a range of keratin filaments may be expressed in various epithelial cells, it is thought that the intermediate filament system of epithelial cells from the small and large intestinal segments *in vivo* contains cytokeratin-18 (CK-18).

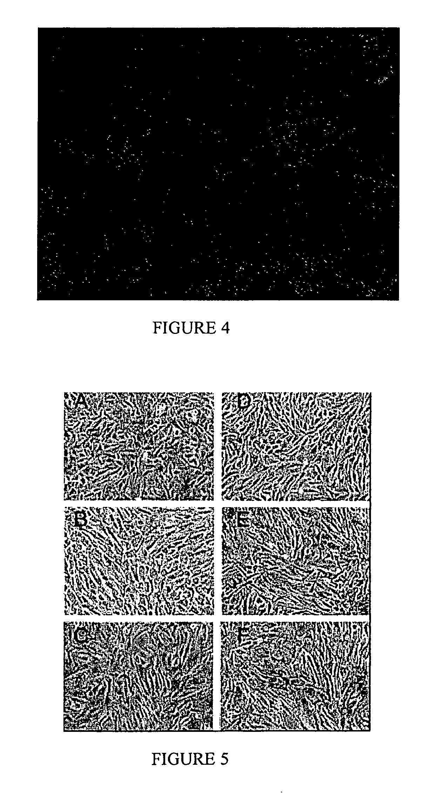

Gastrointestinal epithelial stem-cell-like progenitor cells that were isolated as described in Example 1, were grown on cover slips (Fisher Scientific). After permeabilization and fixation, cells were stained with anti-CK-18 antibody. Cells were dissociated by trypsinization (0.05% Trypsin-EDTA (GIBCO) at 37°C for 2 minutes). Cells then were washed three times in PBS/BSA, fixed in cold methanol at -20°C for 10 minutes, then incubated in 2% BSA/PBS for 5 minutes. A FITC conjugated antibody (1:200 dilution) against anti-human cytokeratin-18 (Sigma, St. Louis, MO) was used to detect the presence of cytokeratin-18. Cells (250,000) were incubated with diluted anti-CK18 antibody (100 µl) for 45 minutes at 4°C, then washed three times with 1% PBS/BSA. Cells suspended in PBS/BSA were placed onto a glass slide, briefly air dried, mounted with a coverslip, then analyzed for the presence of immunofluorescence staining by using an immunofluorescence microscope (Axiovert). **Figure 4** shows a photomicrograph (20X magnification) of a gastrointestinal epithelial stem-cell-like progenitor cell monolayer derived from a jejunal segment stained with anti-CK-18 antibody. An isotope matched control antibody served as negative control antibody which did not show any fluorescence staining giving a dark field on microscopic examination (data not shown). As shown in **Figure 4**, CK-18 was present (green fluorescence) in about 100% cells of the monolayer as confirmed by fluorescence microscopic analysis.

### Example 6. Morphologic Appearance of a Panel of HIPEC Lines Generated From Various Segments of the Intestinal Tract

HIPEC cells were grown in HIPEC-2 medium (66% HGISC medium, 33% MEM, 2% dialyzed FCS, 1% BSA) at 37°C in a humidified CO₂ incubator to encourage differentiation.

**Figure 5** shows light photomicrographs (Zeiss axivert) of representative primary epithelial monolayers derived from gastrointestinal epithelial stem-cell like progenitor cells isolated from the: (A) duodenum, (B) jejunum, (C) ascending colon, (D) transverse colon, (E) sigmoid, and (F) rectum. All of the colonic HIPEC lines were derived from the normal portion of a resected specimen from patients suffering from different disorders.

Monolayers from all colonic HIPEC lines had similar morphology. The HIPEC lines derived from the duodenum and jejunum differed from each other in that the duodenal HIPECs were smaller in size with cuboidal shape while the jejunum HIPECs had a spindle-like shape. Without being limited by theory, these differences in morphology may represent an actual regional difference or may relate to the age or density of the cells in culture.

The HIPEC monolayers are easily detached from the plastic surface and may be subjected to multiple passages (up to 24 passages in cases of normal tissue derived cells). This allows up to 165 x 10⁹ cells in 18-24 passages to be obtained.

A portion of each HIPEC line from each passage was kept frozen in multiple aliquots. The capacity of the frozen HIPEC lines to grow in culture after thawing was tested. The defrosted cells from each passage were capable of growing in secondary cultures (data not shown).

### Example 7. HIPEC Lines are Non-Transformed

Soft agar cultures were prepared as follows: a) 1% agarose solution (5 ml) was added into a 10 cm² petri dish until the plate was completely covered; b) the agarose was pipetted off leaving a thin film of agarose on the bottom of the petri dish; c) after allowing the agarose film to solidify on the plate for 20 minutes with the lid on, 10 ml of cell suspension (0.5 x 10⁶ cells) was added on the top of the agarose film; d) medium was replaced every 4-5 days. 2.0 ml suspensions of normal HIPEC and control HT-29 cells were grown on soft agar medium for 3 weeks. The culture dishes were stained with 0.1% crystal violet for 5 minutes, destained, and photographed.

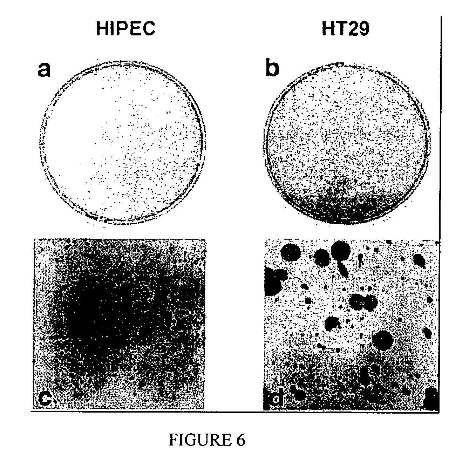

**Figure 6** shows HIPEC lines grown on soft agar, and stained with 0.1 % crystal violet, then destained. It shows that normal colonic HIPEC lines failed to grow in this medium, while foci formation and cell growth was observed in cultures of control malignant colonic epithelial cell line HT-29.

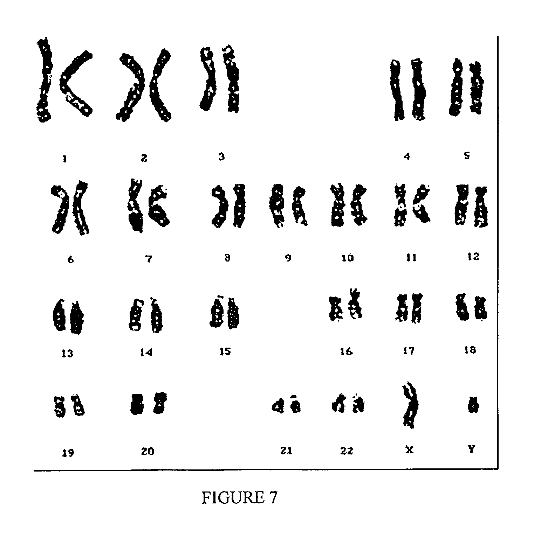

**Figure 7** shows a karyotype analysis of cells from normal colonic HIPEC cells grown for 7 passages. The karyotype analysis of metaphase of the representative cell confirmed a normal 46 XY complement. This data show that the HIPEC lines do not possess a transformed phenotype or malignant nature, and thus are non-transformed.

### Example 8. Differentiation of HIPEC Epithelial Cells

Differentiation of the HIPEC cells was confirmed by microscopy, flow cytometry, or immunofluorescence. The growth conditions described herein allow differentiation of the gastrointestinal stem-cell-like epithelial progenitor cells, of the described invention, to substantially pure populations (meaning greater than about a 85% homologous cell population) of segment-specific epithelial cells.

HIPEC specific markers were analyzed for each epithelial monolayer to confirm its regional identity.

### Example 8.1. Determination of Regional Specific Intestinal Epithelial Enzymatic Characteristics

### Example 8.1.1. Biomarkers: IAP and SI

The intestine-specific enzyme markers human intestinal alkaline phosphatase (IAP) and sucrase isomaltase (SI), combined with immunofluorescence staining, were used for enzyme expression analysis. Samples of HIPEC lines derived from small intestine (jejunum) and large intestine (colon) were treated with anti-human IAP antibodies and anti-human SI antibodies.

Briefly, HIPEC cells were grown in HIPEC-2 medium (66% HGISC medium, 33% MEM, 2% dialyzed FCS, 1% BSA) at 37°C in a humidified CO₂ incubator to encourage differentiation. Expression of intestinal cell specific enzymes were determined by intracellular staining with monoclonal antibodies against intestinal alkaline phosphatase (Dako), and sucrose isomaltase, or an appropriate isotype control antibody. Cells were dissociated with trypsin, then permeabilized and fixed by incubation with permeafix (Ortho, Raritan, NJ) for 1 hour at 25°C, washed twice with PBS/BSA, and resuspended at 4x10⁶ cells/ml. An aliquot of the suspension (25µl (105 cells)) was incubated for 45 minutes on ice with an appropriate amount of antibody (as determined through serial dilution analysis with control cells or as was instructed by the manufacturer) of either of the above-mentioned mAbs or an appropriate isotype control. This was followed by 3 washes with PBS/BSA and subsequent incubation of the cell suspension with FITC conjugated F (ab)'2 goat anti-mouse IgG for an additional 45 minutes. After this second incubation, cells are washed thrice with PBS/BSA, resuspended in 200 µl of PBS and analyzed with FACScan flow cytometer.

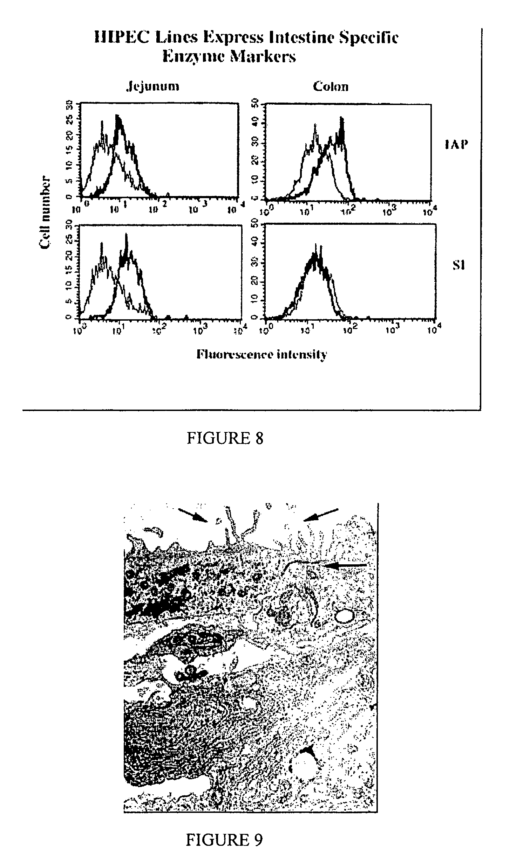

Flowcytometric analysis of the stained cells was performed using a FacScan (Becton Dickinson, San Jose, CA) with the following parameters: (a) light scatter dot plot set up (forward vs. side) was used to gate the homogenous cell populations within the sample; (b) green or red fluorochrome dyes were utilized depending on the antibody used; (c) the controls comprised (i) unstained cells for setting up the cursors for eliminating potential background and (ii) cells stained with isotype matched control antibody to setup the cursor for non-specific fluorochrome intensity; (d) a mean fluorescence channel was used to determine the fluoroescence intensity of the stained cells; and (e) 10,000 cells were gated from a suspension of 1x10⁶ cells/ml. **Figure 8** shows a plot of fluorescence intensity versus cell number. The results presented in **Figure 8** show that the HIPEC lines derived from both small intestine and large intestine were positive for human IAP, whereas the expression of SI was only observed in small bowel HIPEC derived cell lines This enzymatic profile confirmed the region specific intestinal epithelial enzymatic characteristics of the HIPEC lines.

HIPEC cells display ultra-structural features characteristic of differentiated epithelial cells. HIPEC monolayers were grown on nylon tissue culture transwell membranes. These cells were fixed in 4% glutaraldehyde, 0.1 M sodium cacodylate buffer, pH 7.4, post-fixed in 1 % buffered osmium tetroxide, dehydrated in a graded series of ethanol, and infiltrated in LX112 Epon Resin (Ladd, Burlington, VT). Thin sections (70 nm) were picked up on copper formvar coated grids, stained with lead citrate and uranyl acetate and scoped on a Zeiss EM 10 transmission microscope. As shown in **Figure 9****,** electron microscopic examination of the cells reveals structural organization typical of intestinal epithelial cells with numerous microvilli are present on the apical membrane and there is a basement membrane formed at the basal surface of the HIPEC monolayer. The cytoplasm of these cells is filled with bundles of microfilaments and their nuclei are large. A number of coated pits on the cell are visible, suggesting that the cells contain the apparatus for endocytosis typical of epithelial cells.

### Example 8.1.2. Biomarkers: Von Willebrand's Factor, Carcinoembryogenic Antigen, and Secretory Component

The expression by HIPEC lines of biomarkers indicative of region specific intestinal epithelium was analyzed by flowcytometry. The biomarkers included: secretory component (SC), Von Willebrand's Factor (VWF), and carcinoembryogenic antigen (CEA). Secretory component is a component of IgA, a type of antibody that protects against infections of the mucus membranes lining the mouth, airways and digestive tract. VWF is a large multimeric glycoprotein involved in hemostasis present in blood plasma. It is produced constitutively in endothelium, megakaryoctyes and subendothelial connective tissue. CEA is a glycoprotein involved in cell adhesion.

Without being limited by theory, it is thought that the expression of SC and the presence of CEA, and the non-expression of smooth muscle actin is indicative that the cells are not myofibroblasts. Further, SC expression on HIPEC surface is thought to be a characteristic marker of epithelial cell type, as well as being indicative of the differentiating stage of the intestinal epithelium (glandular epithelium). The level of SC expression (high or low) in HIPECs (duodenum through rectum) may indicate the maturity (crypt or surface-like cells) status.

The expression of epithelial cell specific surface molecules (CEA and SC) was assessed by immunofluorescence staining using monoclonal antibodies (purchased from SIGMA and DAKO respectively) against these molecules. An unrelated control antibody served as a negative control. Additionally, to a certain that HIPEC lines are not contaminated with smooth muscle or endothelial cells, a marker for smooth muscle cell (anti-smooth muscle actin) and a marker for endothelial cell (Von -Willebrand Factor (VWF)) also was included in these experiments. Dissociated HIPEC monolayers of six GI tissues (duodenum, jejunum, ascending colon, transverse colon, sigmoid, and rectum) were washed twice in PBS/BSA and then stained by the procedure described above. Stained cell suspensions were analyzed on a flow cytometer (BD) gating on viable cells. Mean channel fluorescence, which correlates with fluorescence intensity, was determined from the peak of positively stained cells and was recorded on a log scale.

**Figure 10** shows plots of fluorescence intensity versus cell number of the cells stained with an anti-CEA (right panel - red lines), anti-VWF (middle panel - green lines), anti-SC (left panel - blue lines), or control antibody (black lines). The flowcytometric analysis of the stained cells was performed as described in Example 8.1.1. For the detection of SC, staining was performed on unpermeabilized cells. All HIPEC lines were positive for secretory component (left panel) and negative for VWF (middle panel) and SMC (data not shown). Levels of CEA expression (middle panel) were segment dependent. The bottom panel row represents control cell lines: the colonic adenocarcinoma cell line HT29 was used to demonstrate both SC (left panel) and CEA (right panel) expression, and a endothelial cell line, HUVEC (center panel) was used to demonstrate VWF expression. These results show that the HIPEC lines express SC, VWF and CEA, indicative that HIPEC lines express biomarkers indicative of region-specific intestinal epithelium.

### Example 8.1.3. Biomarkers: Zonula Occludens, E-cadherin and β-catenin

The generation of regional specific monolayers was confirmed by documenting evidence of region specific protein. A number of cell-cell adhesion molecules and receptors are specific to epithelium. For example, epithelial junctions of the intestine are formed predominantly of zonula occludens (ZO) and/or complexes between E-cadherin and β-catenin. ZO, or tight junctions, are the closely associated areas of two cells whose membranes join together forming a barrier to fluid. β-catenin is an approximate 88 kDa subunit of the cadherin protein complex and is involved in the formation of adherens junctions. E-cadherin (also known as cadherin 1, type 1, CDH1, CD324) is a calcium dependent cell-cell adhesion glycoprotein that has a role in formation of cell-cell contacts in epithelium.

The expression of ZO proteins, E-cadherin and β-catenin in HIPEC monolayers were analyzed by Western blot analysis using specific monoclonal antibodies (Transduction Laboratories) against these junction proteins. For Western blot analysis, the monolayers of HIPEC cells were washed three times in PBS, and lysed by adding Lipper Buffer directly onto the monolayer. Cell lysates were collected and boiled for 10 minutes. Nuclear proteins and debris were pelleted by ultracentrifugation at 10,000 rpm for 10 minutes. The supernatant was analyzed for the content of intestinal epithelial cell specific enzymes and junction proteins. Cell lysate samples (containing 25 µg (for β-catenin), 50 µg (for ZO-1) and 80 µg (for E-cadherin) of protein per lane) were then run on a 7.5% SDS-polyacrylamide gel in reducing condition. After 2 hours of electrophoresis at 200V in Laemmli running buffer, the proteins were then transferred to nitrocellulose by electroblotting at 25 mM Tris, 192 mM glycine and 20% methanol buffer. Proteins in nitrocellulose were detected after 1 hour of blocking in 10% non-fat dry milk. The blot then was incubated with primary antibodies. After overnight incubation, the blot was washed extensively in TBS-Tween followed by incubation with an HRP-conjugated sheep anti-mouse immunoglobulin (Amersham, Piscataway, NJ) for 1 hour. The blot then was washed and signals were detected on autoradiographic film by enhanced chemiluminescence (Amersham).

**Figure 11** shows that intestinal epithelial junction proteins are expressed by HIPEC monolayers. Monolayer of a colonic line was permeabilized and fixed by treatment with permeafix and stained with a mouse anti-human E-cadherin (upper panel) or an anti-human β-catenin mAb (lower panel) or an appropriate negative control (not shown). Note the diffuse staining with anti-β-catenin (both cytoplasmic and surface) as opposed to the punctate staining with anti-E-cadherin antibody only on the cell surface. Control antibodies did not react with these cells.

**Figure 11(A)** shows Western blot analysis of lysates from HIPEC lines representing various segments of the intestine (MJR = rectum, JHS = sigmoid, TDT = transverse colon, AGA = ascending colon, BDJ = jejunum, KPD = duodenum) using anti-β-catenin (upper panel) and anti-ZO-1 (lower panel) antibodies. HeLa (first lane, upper panel), an uterine derived malignant epithelial cell line, served as a positive control for β-catenin, and HT-29 (last lane, lower panel), a colonic adenocarcinoma epithelial cell line, served as a positive control for ZO-1.

Proteins obtained from cell lines HeLa, MJR, JHS, TDT, AGA, BDJ, KPD and HT29 were reacted with antibodies specific for β-catenin, ZO-1 and E-cadherein. It was observed that (i) antibody specific for β-catenin reacted with protein migrating to molecular weight position 92 kD; (ii) antibody specific for ZO-1 reacted with protein migrating to molecular weight position 220 kD, and (iii) antibody specific for E-cadherin reacted with protein migrating to molecular weight position 120 kD. It is concluded that all HIPEC lines expressed both β-catenin and ZO-1.

**Figure 11(B)** shows the results of Western blot analysis of lysates from representative HIPEC lines derived from a normal colon (WT) and small intestine (Jej) (BDJ) using an anti-E-cadherin monoclonal antibody. A lung tumor epithelial cell line A431 served as a positive control. Antibody specific for E-cadherin reacted with a protein migrating to molecular weight 120 kD; to the same position as a protein in a sample of lung tumor epithelial cell line A431. It is concluded that that protein is E-cadherin. Thus, expression of E-cadherin was detected in representative colonic and small intestinal HIPEC lines (a larger amount of protein was loaded than in the experiments described above).

**Figure 11(C)** shows the results of an immunohistochemical analysis of E-cadherin and β-catenin expression on a representative HIPEC line (grown on cover slips). For the purpose of immunocytochemical staining, subconfluent monolayers of HIPEC lines were grown on glass cover slips (Fisher Scientific), washed three times in PBS/BSA and subsequently fixed in cold methanol at -20°C for 10 minutes. The coverslips then were incubated in 2% FCS/PBS for 5 minutes. The primary antibodies against anti-human E-cadherin (Transduction Laboratories, San Diego, CA), β-catenin (Transduction Laboratories, San Diego, CA) or an appropriate isotype-matched control antibody were added and incubated at 4°C for 1 hour. FITC-conjugated goat anti-mouse immunoglobulins were used as secondary antibodies and were incubated for another 1 hour. Between each incubation the coverslips were rinsed with cold PBS, fixed by treatment with permeafix and stained with a mouse anti-human E-cadherin (upper panel), an anti-human β-catenin mAb (lower panel), or an appropriate negative control (anti-human mouse IgG1). Fig. 10C shows the typical staining pattern of both E-cadherin (punctate ring type staining) and β-catenin (defuse staining in the surface and cytoplasm) that was apparent in substantially all cells. The intensity of the β-catenin expression was much greater than that of E-cadherin and is consistent with the Western blot data. Isotype-matched negative controls for antibodies to either of these proteins were non-reactive.

These results suggest HIPEC lines have the ability to form tight junctions and prevent paracellular permeability.

### Example 9. Absorption and Bio-Permeation

According to the described invention, epithelial monolayers representative of segments of the gastrointestinal tract may be used to study the permeability and absorption of therapeutic agents in each segment by analytical means such as, for example, TEER, AUC, NMR, or LC-MS.

### Example 9.1. Transepithelial Electrical Resistance (TEER) of the HIPEC lines

The electrical resistance of native intestinal epithelium *in vivo* is approximately 40-450 ohm/cm² (see Schmitz, H., et al. Gastroenterol. 116:301-9. 1999, for the values of the electrical resistance of native intestinal epithelium *in vivo*).

Transepithelial electrical resistance of HIPEC lines derived from different regions of the GI tract was measured. Briefly, human gastrointestinal stem cell-derived epithelial cells (2x10⁵) from small intestine (A2J1) and colon (5A) were grown on 4 µ pore transwell filters (Costar) and maintained postconfluency in HIPEC medium and the transepithelial electrical resistance (TEER) was measured with an Voltohmmeter (World Precision Instruments, Sarasota, FL) for 12 days. Values were corrected for background resistance, i.e., TEER prior to seeding the cells.

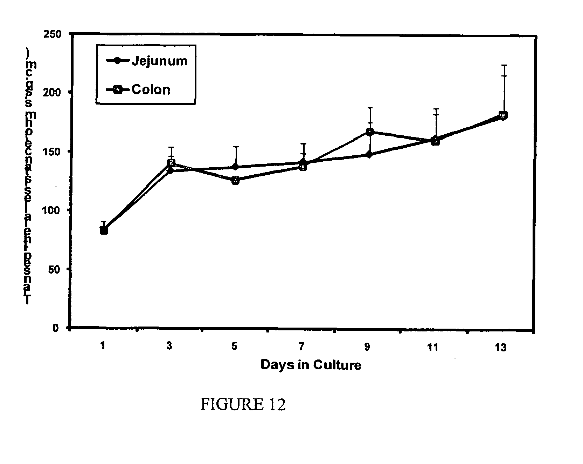

**Figure 12** shows a plot of transepithelial resistance (ohms/cm²) versus the number of days in culture. The error bars represent standard deviation from the mean, n = 3 (for each time point). The average TEER in both the small intestinal (A2J1) and the colonic (5A) cell manolayers was comparable to known physiological (in vivo) TEER, ranging between 150-200 Ohm/cm².

### Example 9.2. Use of HIPEC Monolayer as a System to Assess Drug Permeability and Region Specific Absorption

A panel of at least two FDA suggested drugs (Naproxen, Propanolol), for use in establishing suitability of a permeability method was purchased from USP Reference Standard (Rockville, MD) and used for determination of drug permeability through HGI-SC derived epithelial monolayers from each segment of the GI tract. Primary stock solution of both compounds were prepared in phosphate free PF-DMEM to obtain a concentration of 2 mg/ml of each compound. The stock was diluted to 250 µg/ml to make a working solution and standards for calibration curves and quality control samples were prepared using serial dilutions of the working solution in the PF-DMEM. The concentration range for working standard solutions was from 1.2-50 µg/ml. Drug permeation studies were performed using established methods specific for each drug. Briefly, 2x10⁵ cells were seeded on a transwell membrane (Costar) to form a polarized monolayer and then a drug (50 mM/ml) was added on the apical chamber and incubated at 37°C for various lengths of time. Samples from the bottom chamber were analyzed for the amount of drug that permeated through the monolayer by utilizing a LC/MS spectrometer at Sannova Analytical Inc, a FDA approved analytical laboratory. Briefly, the samples were extracted using an acetonitrile precipitation method and analyzed by using reverse-phase liquid chromatography (Shimadzu, Kyoto, Japan) and tandem MS detection with an API4000 LC/MS/MS system (Forester City, CA) for subsequent analyte/drug compound detection.

### Example 9.2.1. Naproxen Permeability Through Epithelial Monolayers Derived from Human Gastrointestinal Epithelial Stem Cell-Like Progenitor Cells

The permeability of a therapeutic agent, naproxan, through epithelial monolayers derived from human GI stem cells ("HGI-SC") was analyzed. Naproxen is a nonsteroidal antiinflammatory drug used in the treatment of arthritis and musculoskeletal pain. Briefly, representative HGI-SC derived epithelial cells from 10 segments of the GI tract were cultured on transwell filters (4 µm pore size) (Costar) placed in a 24 well plate, with daily replacement of the culture medium. This setup comprises a transwell filter containing HGISC cells interspersed between the donor and recipient chambers. These segments included: (1) esophagus; (2) stomach; (3) duodenum; (4) jejunum; (5) ileum; (6) cecum; (7) ascending colon; (8) transverse colon; (9) sigmoid; and (10) rectum. Upon reaching confluency the monolayers on the transwells were forced to polarize by adding HIPEC-1 medium (0.7 ml) (i.e., HGISC medium without autologous growth factor but + 2% FCS) on the upper chamber (apical side of the monolayer) and RPMI 1640 (1 ml) (free of any growth factor or serum) only at the bottom chamber (basal side). The medium in both chambers were refreshed daily. On day 8, the monolayers as well as the basal chambers were gently washed with phosphate free DMEM (phosphate interfares in LC/MS analysis) and Naproxan (50 µM) dissolved in phosphate free DMEM medium was added to the upper chamber (on the top of the monolayer - the donor chamber) of the transwell membrane. The bottom chamber (recipient) contained an equal volume of medium without the naproxan additive. Samples from the recipient chamber were collected at 24 hours and 48 hours. These samples were analyzed by mass spectrometry analysis (GC-MS/MS) for their content of naproxen absorbed from the apical (upper) surface and transported to the basal compartment by the HGISC derived non-transformed epithelial monolayers. The donor chamber also was analyzed after 48 hours to determine the presence of untransported drug.

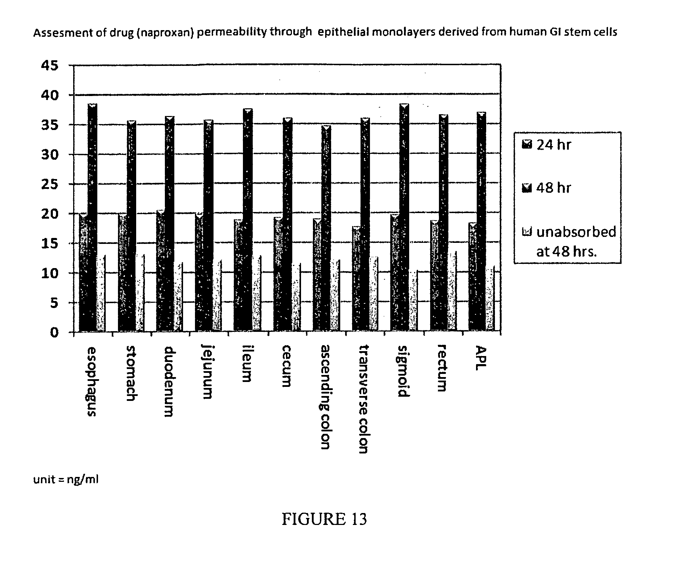

**Figure 13** shows a bar graph illustrating the amount of drug (naproxan) (ng/ml) absorbed or unabsorbed through epithelial monolayers derived from human GI stem cells. **Figure 13** shows that Naxopran was detected in the recipient chambers below the epithelial monolayers for all the epithelial monolayers at 24 hours after the addition of the therapeutic agent to the culture medium. This amount increased approximately 2-fold after 48 hours. These results demonstrate that the therapeutic agent Naxopran is permeable through the epithelial monolayers derived from HGI-SC.

### Example 9.2.2. Propranolol Permeability Through Epithelial Monolayers Derived from Human Gastrointestinal Epithelial Stem Cell-Like Progenitor Cells

For this example, drug permeability and region specific absorption was demonstrated using propranolol. Propranolol is known to be highly lipophilic, non-selective beta blocker which blocks the action of epinephrine on both β1- and β2-adrenergic receptors. When administered *in vivo,* propranolol is absorbed rapidly and completely with peak plasma levels achieved approximately 1 to 3 hours after ingestion. Without being limited by theory, coadministration with food appears to enhance bioavailability. Despite complete absorption, propranolol has a variable bioavailability due to extensive first-pass metabolism. Hepatic impairment therefore will increase its bioavailability.

The absorption and bio-permeation of propanolol was determined for representative monolayers derived from the gastrointestinal epithelial stem-cell like progenitor cells of the described invention from large (5A) and small intestine (A2J1). The basal level electrical resistance of the HIPEC lines was determined prior to plating of the cells. Electrical resistance levels initially increase after plating of the cells as the cells multiply, migrate and make contact with one another, forming junctional complexes. The observed TEER level is maintainable for approximately 10 days after the cells representative of a colon derived HIPEC (5A) and jejunum derived cells (A2J1) have reached confluency (see **Figure 12**).

The donor compartments of the colon derived line (5A) and small intestine derived line (A2J1) were initially loaded with 50 ug/ml of propanolol and samples removed from both the donor and recipient compartments at 0, 30, 60, 90, and 180 minute time intervals. The samples were analyzed by tandem LC-MS utilizing a Waters' XBridge C18 column on a Shimatzu 20AD HPLC coupled with an Applied BioSystem ABI4000 mass spectrometer and the observed relative values entered into the table. **Table 1** shows the results of the assessment of propranolol using HIPEC monolayers derived from colon (5A) and small intestinal (A2J1) tissue segments. Propanolol was able to both exit and to be absorbed by both cell lines; decreasing in the donor compartment and accumulating in the recipient compartment over time. **Table 1** shows that the amount of propranolol in the donor compartment decreased over time and the amount in the recipient compartment increased over time. The difference is attributed to cell absorption of propranolol. The data demonstrated that propranolol moved into the recipient compartment of A2J1 cells faster than of 5A cells. Therefore one may conclude that the data demonstrated propanolol permeability through small intestine (A2J1) is greater than through large intestine (5A).

**Table 1 Drug Permeability Assessment and Prediction of Region Specific Absorption by Using Representative HIPEC Monolayers Derived from Colon (5A) and Small Intestinal Segments (A2J1)**

| **Time (mins.)** | **5A-recipient compartment** | **A2J1-recipient compartment** | **5A-donor compartment** | **A2J1-donor compartment** |
|---|---|---|---|---|
| **0** | **0.00** | **0.00** | **50.00** | **50.00** |
| **30** | **4.25** | **4.50** | **30.84** | **33.47** |
| **60** | **7.28** | **7.79** | **23.40** | **26.84** |
| **90** | **9.67** | **10.49** | **20.00** | **20.54** |
| **180** | **11.02** | **12.44** | **17.70** | **17.53** |

These results indicate the HIPEC lines of the described invention can provide a system for analysis of drug permeability.

### Example 10. Stem Cells Isolated from Adult Human Small Intestinal Tissue

Gastrointestinal epithelial stem-cell like progenitor cells were isolated. The isolated stem cells were identified with analysis of (i) the expression of known stem cell markers; (ii) self-renewal; and (iii) pluripotency.

### Example 10.1. Stem Cell Marker Expression of the Cultured Stem Cells

### Example 10.1(a). Amplification of Biomarkers: Protocols and Primers

The protocols and primers utilized in the following Examples were as follows:
PCR amplification products from Nanog (852 bp), LIN28 (828 bp) and Oct4 (variant 1) (828 bp) were obtained utilizing 35 cycles of the following protocol: (i) 94°C for 40 seconds, (ii) 56°C for 30 seconds; (iii) 73°C for 1 minute.
PCR amplification products from Nanog (852 bp) were obtained utilizing the following primers: (forward) ATGCCTGTGATTTGTGGGCC [SEQ ID NO: 1] and (reverse) CTCATCTTCACACGTCTTCAGGTTG [SEQ ID NO: 2].
PCR amplification products from LIN28 were obtained utilizing the following primers: (forward) CAACCAGCAGTTTGCAGGTGGCTG [SEQ ID NO: 3] and (reverse) GAACCCTCACTTGCATTTGGACAGAG [SEQ ID NO: 4].
PCR amplification products from Oct4 (variant 1) were obtained utilizing the following primers: (forward) CGGGACACCTGGCTTCGGATTTCG [SEQ ID NO: 5] and (reverse) CTTGTAAGAACATAAACACACCAG [SEQ ID NO: 6].
PCR amplification products from Oct4 (variant 1 and 2) (455 bp) and β-tubulin (385 bp) were obtained utilizing 30 cycles of the following protocol: (i) 94°C for 40 seconds, (ii) 56°C for 30 seconds; (iii) 73°C for 45 seconds.
PCR amplification products from Oct4 (variant 1 and 2) were obtained utilizing the following primers: (forward) CATCAAAGCTCTGCAGAAAGAACTC [SEQ ID NO: 7] and (reverse) CTGCTTGATCGCTTGCCCTTCTGGC [SEQ ID NO: 8].
PCR amplification products from β-tubulin were obtained utilizing the following primers: (forward) CTTGTAAGAACATAAACACACCAG [SEQ ID NO: 9] and (reverse) CTGGAGGCTTAGGGACCAAGGCTG [SEQ ID NO: 10].
PCR amplification products from Oct4 (variant 2) (471 bp), Bmi1 (576 bp) and LGR5 (498 bp) were obtained utilizing 40 cycles of the following protocol: (i) 94°C for 40 seconds, (ii) 56°C for 30 seconds; (iii) 73°C for 45 seconds.
PCR amplification products from Oct4 (variant 2) were obtained utilizing the following primers: (forward) CATGAGTCAGTGAACAGGGAATG [SEQ ID NO: 11] and (reverse) GGTTTCTGCTTTGCATATCTCCTG [SEQ ID NO: 12].
PCR amplification products from Bmi 1 were obtained utilizing the following primers: (forward) CATAATAGAATGTCTACATTCCTTCTG [SEQ ID NO: 13] and (reverse) GGAAGTGGACCATTCCTTCTCCAG [SEQ ID NO: 14].
PCR amplification products from LGR5 were obtained utilizing the following primers: (forward) GATCTGTCTTACAACCTATTAGAAG [SEQ ID NO: 15] and (reverse) CTTCAAGGTCACGTTCATCTTGAGC [SEQ ID NO: 16].
PCR amplification products of two different lengths were obtained from SOX2 (581 bp and 621 bp) utilizing 35 cycles of the following protocol: (i) 94°C for 40 seconds, (ii) 56°C for 30 seconds; (iii) 73°C for 45 seconds.
PCR amplification products from SOX2 (581 bp) were obtained utilizing the following primers: (forward) CAAAAGTCTTTACCAATAATATTTAGAG [SEQ ID NO: 17] and (reverse) GCCGAATCTTTTAAAATACAACTACG [SEQ ID NO: 18].
PCR amplification products from SOX2 (621 bp) were obtained utilizing the following primers: (forward) TAAAAGTTCTAGTGGTACGGTAGGAG [SEQ ID NO: 19] and (reverse) GCCGAATCTTTTAAAATACAACTACG [SEQ ID NO: 20].

### Example 10.1(b). Expression of Stem Cell Markers by Cultured Gastrointestinal Epithelial Stem Cell-like Progenitor Cells

Cultured gastrointestinal epithelial stem cell-like progenitor cells were analyzed for the presence of the stem cell markers Nanog, Oct4, LIN28, SOX2 and the putative intestinal stem cell markers Lgr5 and Bmi-1.

Stem cell markers: (a) β-tublin (385 bp); (b) Nanog (852 bp); (c) LIN28 (829 bp); (d) Oct4 (variant 1 and 2; 455 bp); (e) Oct4 (variant 2; 471 bp); (f) Oct4 (variant 1; 828 bp); (g) SOX2 (581 bp); (h) Bmi1 (576 bp); (i) Lgr5 (498 bp).

β-tubulin is a 55 kD globular protein component of microtubules. It is believed that the expression of β-tubulin generally is unaffected by experimental conditions, thus β-tubulin frequently is used as a positive control.

Nanog is a transcription factor involved with self-renewal of undifferentiated embryonic stem cells. This 305 amino acid protein is thought to have a key role in maintaining pluripotency, and thus is frequently used as a pluripotency marker. It is further thought that the loss of Nanog function causes differentiation of embryonic stem cells into other cell types.

Octamer-4 ("Oct-4") is a homeodomain transcription factor of the POU family. This protein is involved in the self-renewal of undifferentiated embryonic stem cells. It is thought that Oct-4 expression is associated with an undifferentiated phenotype. As such, it is frequently used as a marker for undifferentiated cells.

LIN-28 homolog ("LIN28") is a marker of undifferentiated human embryonic stem cells. LIN28 encodes a cytoplasmic mRNA-binding protein that binds to and enhances the translation of lgf2 mRNA. It has been used to enhance the efficiency of the formation of induced pluripotent stem cells from human fibroblasts.

SRY (sex determining region Y)-box 2 ("SOX2") is a transcription factor that is involved in maintaining self-renewal of undifferentiated embryonic stem cells. It is thought that SOX2, in induced pluripotent stem cells, is involved in the regulation of Oct-4. Thus, SOX-2 frequently is used as a marker for self-renewal of undifferentiated embryonic stem cells.

Leucine-rich repeat-containing G protein-coupled receptor 5 ("LGR5") is a putative stem cell marker of the intestinal epithelium. In the intestine, Lgr5 is thought to be exclusively expressed in cycling crypt base columnar cells. These crypt base columnar cells are capable of self-renewal and multipotency, thus may represent genuine intestinal stem cells. As such, Lgr5 is thought to represent a marker for adult stem cells.

BMI1 polycomb ring finger oncogene ("BMI1") is thought to be an oncogene in humans and necessary for efficient self-renewing cells divisions of adult hematopoietic stem cells, as well as adult peripheral and central nervous system neural stem cells. As such, Bmi 1 may represent a marker for intestinal stem cells.

Total RNA was prepared from stem cell lines obtained from 10 regions (tissues) of the GI tract as described in Example 3. The stem cell lines analyzed included: (1) stem cell line control (human embryonic stem cell line RNA); (2) human dermal fibroblast cell line control ("HDF") (Cell Applications Inc., San Diego, CA); (3) peripheral blood monocyte control (freshly isolated from peripheral blood from a healthy donor); (4) esophagus derived stem cell line D1708E; (5) gastric derived stem cell line D1708G; (6) duodenum derived stem cell line D1708D; (7) jejunum derived stem cell line D1708J; (8) ileum derived stem cell line D1708I; (9) cecum derived stem cell line D1708C; (10) ascending colon derived stem cell line D1708A; (11) transverse colon derived stem cell line D1708T; (12) sigmoid derived stem cell line D1708S; and (13) rectum derived stem cell line D1708R.The total RNA obtained from each gastrointestinal epithelial stem cell-like progenitor cell line then was used to provide a template for RT-PCR, as described in Example 3. All RT-PCR reactions were performed with equal amounts of total RNA (2 µg/RT reaction).

**Figure 14** shows agarose gel patterns of the amplification products (10 µl from the 50 µl PCR reaction solution) acquired from RT-PCR utilizing each of the total RNA templates obtained from the gastrointestinal epithelial stem cell-like progenitor cells. **Figure 14** shows (i) each gastrointestinal epithelial stem cell-like progenitor cell line, including: Lane (M) a 100 bp DNA molecular weight marker (VWR, Catalog No. 95039-808); (1) stem cell line control; (2) human dermal fibroblast cell line control; (3) peripheral blood monocyte control; (4) esophagus derived stem cell line D1708E; (5) gastric derived stem cell line D1708G; (6) duodenum derived stem cell line D1708D; (7) jejunum derived stem cell line D1708J; (8) ileum derived stem cell line D1708I; (9) cecum derived stem cell line D1708C; (10) ascending colon derived stem cell line D1708A; (11) transverse colon derived stem cell line D1708T; (12) sigmoid derived stem cell line D1708S; and (13) rectum derived stem cell line D1708R; and (ii) several biomarkers, including: (a) β-tublin (385 bp); (b) Nanog (852 bp); (c) LIN28 (829 bp); (d) Oct4 (variant 1 and 2; 455 bp); (e) Oct4 (variant 2; 471 bp); (f) Oct4 (variant 1; 828 bp); (g) SOX2 (581 bp); (h) Bmi1 (576 bp); (i) Lgr5 (498 bp).

Based on this analysis, all of the cultured cell lines were positive for the stem cell markers Nanog (row 6), Oct4 (rows d-f), LIN28 (c), SOX2 (row g), and the putative intestinal stem cell marker Bmi 1 (row h).

**Figure 14** further shows that the total RNA obtained from the cultured cell lines from the duodenum (row i, lane 6), jejunum (row i, lane 7) and transverse colon (row i, lane 11) were negative for Lgr5 expression. Positive Lgr5 expression was observed with cultured cell lines from the esophagus (row i, lane 4), gastric/stomach (row i, lane 5), ileum (row i, lane 8), cecum (row i, lane 9), ascending colon (row i, lane 10), sigmoid (row i, lane 12), and rectum (row i, lane 13). A comparison of the amplification products from the Lgr5 marker and β-tublin (as a positive control) obtained from the total RNA of these stem cell lines suggests that the putative intestinal stem cell marker Lgr5 is selectively expressed.

### Example 10.2. Self-renewal Capability of the Cultured Stem Cells

### Example 10.2.1. Self-renewal Capability of Small Intestine Stem Cell Lines

Briefly, small intestine stem cell lines were cultured for 10 passages. Each time the surface of a culture became confluent, cells were split in a 1:3 ratio and each splitting was considered "one passage", thus 10 passages represent approximately 40 cellular divisions at 37°C in a humidified 5% CO₂ supplemented incubator in HIPEC-1 medium. A stem cell line (A2J1) obtained from a section of the jejunum was allowed to undergo 10 passages, then processed to obtain total RNA, as described in Example 3. This total RNA then was analyzed utilizing RT-PCR for the presence of the stem cell markers Oct4 and Nanog, as well as β-tubulin (positive control), as described in Example 3. As in Example 3, an equal amount of initial total RNA template was used; the detection of β-tubulin from the total RNA template was used as a positive control.

**Figure 15** shows amplification products of Oct4, Nanog and β-tubulin during each of the 10 serial passages of the A2J1 stem cell line. These results demonstrate that for at least 10 passages the cultured cell line A2J1 (i) expressed stem cell markers, (ii) maintained its self-renewal capability, and (iii) remained non-differentiated. If the cells were fully differentiated to a specific cell type (such as the fibroblast cell line (HDF) used as a differentiated non-stem cell control cell line in Figure 13), then one would expect these cells to loose the stem cell markers (i.e., Nanog gene expression).

### Example 10.2.2. Self-renewal Capability of Gastrointestinal Epithelial Stem Cell-Like Progenitor Cell Lines

Cell lines prepared from the duodenum, jejunum, ileum, ascending colon, transverse colon, sigmoid, rectum, and jejunum in HIPEC-1 were cultured for 10 passages.). HIPEC-1 medium was utilized to effects on differentiation. Total RNA from each of the cell lines was collected, as described in Example 3, during selected passages. These collections included the total RNA from the: (a) First passage of cell lines cultured from the ileum and ascending colon; (b) Second passage of the cell line cultured from the jejunum; (c) Fourth passage of the cell line cultured from the sigmoid; (d) Sixth passage of the cell lines cultured from the jejunum, ascending colon, rectum, and jejunum in HIPEC-1; (e) Seventh passage of the cell line cultured from the duodenum; (f) Eighth passage of the cell line cultured from the sigmoid; and (g) Ninth passage of the cell line cultured from the transverse colon. Each of the total RNA collected then was separately analyzed for the presence of the stem cell markers Oct4, Nanog and LIN28, as well as β-tubulin (postive control), utilizing RT-PCR.

As in Example 10.1, an equal amount of initial total RNA template was used.

**Figure 16** shows the RT-PCR products from total RNA preparation of the cell lines derived from the duodenum (lane 2), jejunum (lane 3), ileum (lane 4), ascending colon (lane 5), transverse colon (lane 6), sigmoid (lane 7), rectum (lane 8), jejunum (lane 9), jejunum in HIPEC-1 (lane 10), jejunum in HIPEC-1 (lane 11), jejunum (lane 12), ascending colon (lane 13) and sigmoid (lane 14). It reveals that (i) all of the cell lines expressed the stem cell marker Oct4 although the level of expression was not uniform for all; (ii) the cell lines from the duodenum (lane 2), ileum (lane 4), sigmoid (lane 7), rectum (lane 8), jejunum (lane 9), ascending colon (lane 5), and jejunum in HIPEC-1 (lanes 10,11) expressed the stem cell marker Nanog; and (iii) the cell lines from the duodenum (lane 2), ileum (lane 4) and rectum (lane 8) expressed the stem cell marker LIN28. All cell lines expressed the β-tubulin positive control. These results show that primary cell lines cultured from different segments of the gastrointestinal tract can be passaged and retain their ability to express stem cell markers.

### Example 10.3. Pluripotency of the Cultured Stem Cells

Pluripotent gastrointestinal epithelial stem cell-like progenitor cells may differentiate to any of four main epithelial cell lineages of the gastrointestinal tract: (i) columnar Epithelial cells, (ii) goblet cells, (iii) enteroendocrine chromaffin cells, and (iv) Paneth cells. Expression of the following molecular markers was determined, as in Example 3 and Example 10.1 (a) and used to identify these differentiated cell types, for example, columnar epithelial cells were identified by the presence of the markers intestinal alkaline phosphatase (ALP1) and sucrase isomaltase (SI); goblet cells were identified by the presence of the markers mucin-2 (MUC2) and trefoil factor 3 (TFF3); enteroendocrine chromaffin cells were identified by the presence of the marker chrmogranin A (CHGA); and Paneth cells were identified by the presence of the markers lysozyme (LYZ) and defensin (DEFA5). The RT-PCR products were identified by the single band generated using primers specific for each molecular marker at the expected molecular weight of the product.

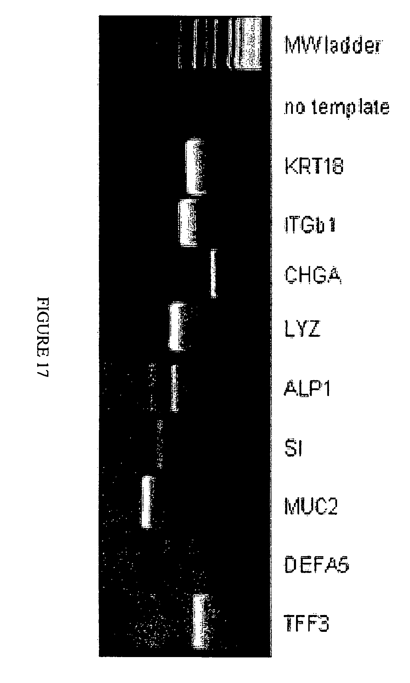

**Figure 17** shows the RT-PCR products of a total RNA preparation from cell line A2J1, which is of jejunum origin. This cell line demonstrated expression of the epithelial markers cytokeratin-18 (KRT18), β1-integrin (ITGb1), chromogranin A (CHGA), lysozyme (LYZ), intestinal alkaline phosphatase (ALP1), sucrase isomaltase (SI), mucin-2 (MUC2), defensin-5 (DEFA5) and trefoil factor 3 (TFF3).

These results demonstrate that the stem cell line A2J1, cultured from the jejunum, underwent differentiation to a mature cell phenotype and, as a result, expressed molecular markers characteristic of each of the four main epithelial cell lineages of the gastrointestinal tract.

### Example 10.3.1. Epithelial Lineage Specific Protein Expression

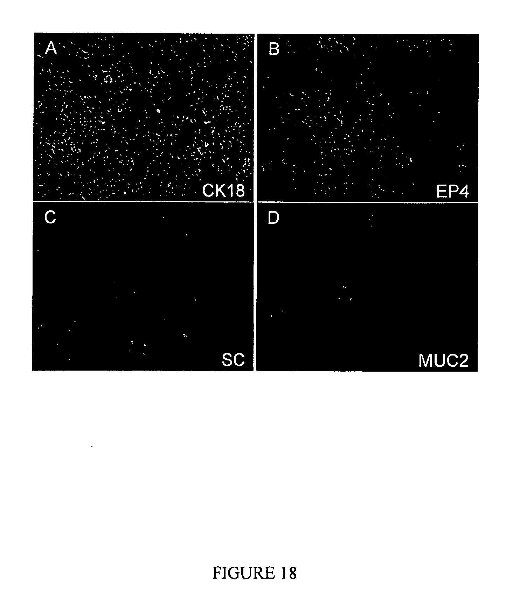

The expression of epithelial lineage specific proteins was analyzed. Briefly, cells from HIPEC lines were immunocytochemical chemical stained. **Figure 18** shows immunofluorescence micrographs of cells immunochemically stained with antibody, as described in Example 8, for CK18, EP4, SC, and MUC2. Enriched (tear drop-like shaped cells) subpopulation HIPEC lines were grown on glass cover slips (Fisher Scientific), washed three times in PBS/BSA and subsequently fixed in cold methanol at -20°C for 30 minutes. The coverslips then were incubated in 2% FCS/PBS for 5 minutes. The primary mouse monoclonal antibodies (IgG1) against human cytokeratin-18 (Sigma), MUC2 (Pharmingen), EP4 (Dako, Denmark) and rabbit anti-human SC (Dako, Denmark), or an appropriate isotype-matched control antibody (mouse IgG1 for CK18, EP4, and MUC2 and rabbit IgG for SC) were added and incubated at 4°C for 1 hour. FITC-conjugated goat anti-mouse immunoglobulin for MUC2 and a goat-anti-rabbit IgG for SC were used as secondary antibodies and were incubated for another 1 hour. Between each incubation the coverslips were rinsed with cold PBS. Anitbodies against CK18 and EP4 were directly FITC conjugated and no incubation with secondary antibodies were required.

As shown in **Figure 18****,** cells were positive for cytokeratin-18 (CK18) and epithelial protein 4 (EP4). A smaller subset of cells were positive for Goblet cell specific secretory component (SC) and mucin-2 (MUC2). These results show that cells from the HIPEC lines expressed epithelial lineage specific proteins and illustrates that the human differentiable segment-specific gastrointestinal epithelial stem-cell-like progenitor cells have the potential to differentiate into a mature cell phenotype.

### Example 10.3.2. Ultrastructure of Columnar Monolayers

Columnar epithelia are epithelial cells whose heights are at least four times their width. Columnar epithelial are divided into simple (unilayered) and stratified (multi-layered).

Goblet cells are glandular simple columnar epithelial cells whose sole function is to secrete mucus. They use both apocrine and merocrine methods for secretion.

Microvilli are microscopic cellular membrane protrusions that increase the surface area of cells, and are involved in a wide variety of functions, including absorption, secretion, cellular adhesion, and mechanotransduction. Thousands of microvilli form a structure called the brush border that is found on the apical surface of some epithelial cells, such as the small intestinal enterocyte and the kidney proximal tubule.

The basement membrane is a sheet of cells and fibers that covers two other kinds of cells -- the epithelium, which lines the cavities and surfaces of organs, and the endothelium, which lines the interior surface of blood vessels. The basement membrane is the fusion of two basal laminae. It consists of an electron-dense membrane called the lamina densa, about 30-70 nanometers in thickness, and an underlying network of reticular collagen (type III) fibrils (its precursor is fibroblasts) which average 30 nanometers in diameter and 0.1-2 micrometers in thickness.

The intracellular lumen is the inside space of a cellular component or structure.

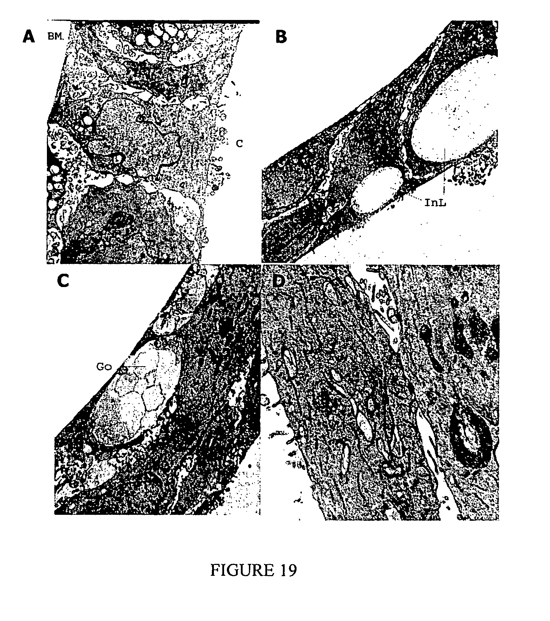

Confluent HIPEC monolayers were grown on biosimilar matrix coated transwell membranes. These cells were fixed in 3% glutaraldehyde in 0.1 M PBS, pH 7.2, at 4oC for 1 hour, then post-fixed in 1% osmium tetroxide, dehydrated in a graded series of ethanol, and infiltrated in LX112 Epon Resin (Ladd, Burlington, VT). Thin sections (70 nm) were picked up on copper formvar coated grids, stained with lead citrate and uranyl acetate and scoped on a Zeiss EM10 transmission microscope. **Figure 19** shows electron microscopy images of the A2J1 (jejunal) cell line. Several structures characteristic of columnar monolayers and Goblet cells were observed, including microvilli, lipid, basement membrane **(****Figure 19A****),** columnar cells **(****Figure 19A****),** intracellular lumen **(****Figure 19B****),** Goblet cells **(****Figure 19C****),** and tight junctions (ZO-1) **(Figure 19D).** These images show that AJ21 cells may differentiate into columnar epithelial cells, and Goblet cells.

### Example 10.3.3. Polarization Ability of HIPEC Lines

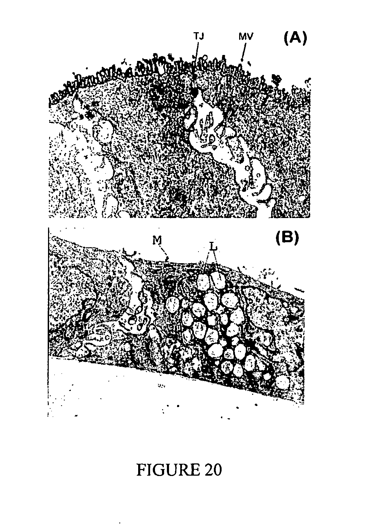

The ability of HIPEC cells to polarize and represent a true model for transport studies of materials, such as, but not limited to, water-electrolyte, nutrients, and oral medicines, was demonstrated by the presence of ultrastructural features characteristic of epithelial cells in HIPEC lines. The presence of these ultrastructural features characteristic of epithelial cells in HIPEC lines was examined utilizing electron microscopy, as described in Example 10.2. The HIPEC line was cultured for 4 passages and were examined by electron microscopy to determine if the cells had acquired characteristics of a mature cell phenotype. **Figure 20** shows electron micrographs of the epithelial cells derived from stem cells from oral mucosal ("APL"). **Figure 20A** shows that the APL cells after 4 passages in differentiation medium contain the following ultrastructural features: (a) microvilli (MV) on the apical surface; and (b) intercellular tight junction (TJ). APL cells are a primary epithelial cell line differentiated from oral mucosa. **Figure 20B** shows that the A2J1 cultures (jejunal cells) contain Goblet cells identifiable by the scant presence of MV and numerous mucin-containing vesicles (L).

These electron micrographs show structural features typical of intestinal epithelial cells: numerous MV present on the apical membrane; the cytoplasm of these cells is filled with bundles of microfilaments; their nuclei are large; and tight junctions (TJ) are present.

### Example 10.3.4. Effect of Growth Media on Differentiation

Several media components and culturing conditions were examined for (i) the ability to propagate stem cells under long term culturing conditions, and (ii) differentiation into a mature cell phenotype. Accordingly, the expression of specific stem cell markers and epithelial cell type specific differentiation markers were examined.

### Example 10.3.4.1. Expression of Enterocyte Epithelial Marker Sucrase Isomaltase in HIPEC Lines Established from Different Gastrointestinal Tissues.

The expression of the epithelial marker sucrase isomaltase (SI) in HIPEC lines established from different gastrointestinal tissues was analyzed. Cell lines from the duodenum, jejunum (lane 3 and 12), ileum (lane 4), ascending colon (lanes 5 and 13), transverse colon (lane 6), sigmoid (lanes 7 and 14), rectum (lane 8), and jejunum in HIPEC-1 (lanes 10-11) were cultured for 10 passages. Total RNA during selected passages of each of the cell lines was collected, as described in Example 3. These collections included the total RNA from the: (a) first passage of cell lines cultured from the ileum and ascending colon; (b) second passage of the cell line cultured from the jejunum; (c) fourth passage of the cell line cultured from the sigmoid; (d) sixth passage of the cell lines cultured from the jejunum, ascending colon, rectum, and jejunum in HIPEC-1; (e) seventh passage of the cell line cultured from the duodenum; (f) eigth passage of the cell line cultured from the sigmoid; and (g) ninth passage of the cell line cultured from the transverse colon. Each total RNA obtained then was separately analyzed for the presence of the stem cell markers Oct4, Nanog and LIN28, as well as β-tubulin (postive control), utilizing RT-PCR. As in Example 10.1, an equal amount of initial total RNA template was used.

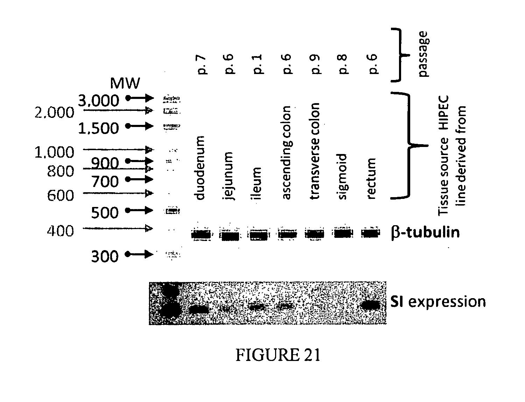

**Figure 21** shows the RT-PCR products from total RNA preparation of each of the cell lines. **Figure 21** reveals that the HIPEC cell lines from the duodenum (lane 2), jejunum (lane 3), ileum (lane 4), ascending colon (lanes 5), transverse colon (lane 6), rectum (lane 8), and sigmoid (lane 7) expressed the SI enterocyte epithelial marker. All cell lines expressed the β-tubulin positive control. These results show that the expression of SI differs amongst cell lines cultured from different segments of the gastrointestinal tract.

### Example 10.3.5. Expression of Vimentin and Cytokeratin-18

The expression of vimentin and of cytokeratin-18 by the HIPEC lines was examined to determine whether human segment-specific gastrointestinal epithelial stem-cell-like progenitor cells may differentiate to a mesenchymal cell. Cells were analyzed for the expression of vimentin, a marker for mesenchymal origin, and cytokeratin-18, a maker for epithelial origin.

Briefly, HIPEC cell lines derived from a colonic stem cell derived primary epithelial cell population were cultured HGISC medium at 37°C in 5% CO₂ supplemented humidified incubator and tested for the expression of epithelial lineage marker protein cytokeratin-18 or the mesenchymal lineage marker vimentin. The expression level of both markers were follwed by immunofluorescence staining with monoclonal antibiodies (Sigma) against vimentin (red) and cytokeratin-18 (green) upto 18 passages.

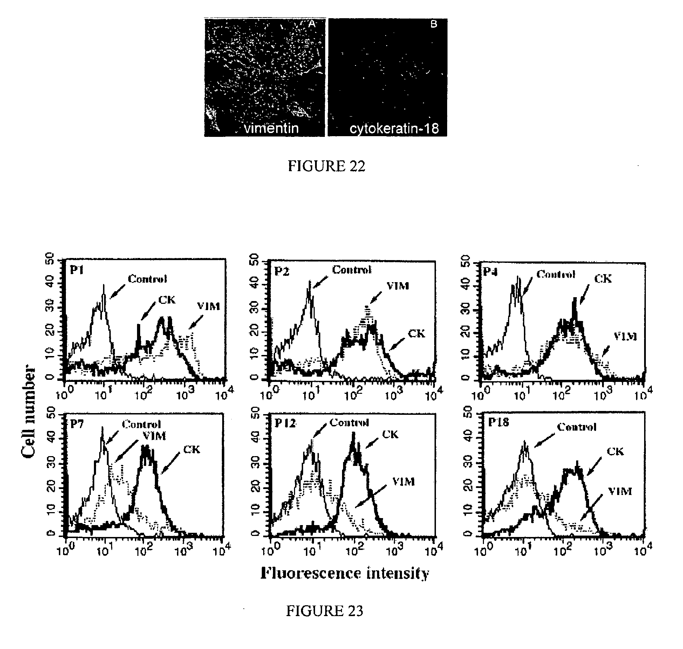

**Figure 22** shows immunochemical staining of HIPEC lines with antibodies against vimentin (panel A, red) and cytokeratin-18 (panel B, green). Subconfluent monolayers of colonic stem cell derived primary epithelial cells were grown on glass coverslips, washed three times in PBS/BSA and subsequently fixed in cold methanol at -20°C for 10 minutes. The cover slips were then incubated in 2% FCS/PBS for 5 minutes. The primary antibodies against anti human cytokeratin-18 or vimentin (Sigma) or an appropriate isotype matched control antibody were added and incubated at 4°C for 1 hour. FITC conjugated (for vimentin) and a phycoerythrine (PE) conjugated goat anti mouse immunoglobulins were used as secondary antibody and were incubated for another 1 hour. Between each incubation the coverslips were rinsed 3 times with cold PBS. The staining intensity was then visualized using a fluorescence microscope (Nikon).

**Figure 22** shows that both of these lineage markers are present between passages 2 and 3 of the HIPEC lines, suggesting that these early cells possess a pluripotent nature and may be capable of differentiating to a mesenchymal cell phenotype.

**Figure 23** shows the results of flow cytometric analysis of epithelial marker cytokeratin-18 (CK; red) and mesenchymal marker vimentin (VIM; blue) expression on HIPECs at various time points: passages 1 (p1); 2 (p2), 4 (p4), 7 (p7), 12 (p12) and 18 (p18). The flow cytometric analysis was performed as described in Example 8. The results show that expression of VIM gradually disappeared as cell growth and differentation progressed while CK-18 expression remained unchanged through 18 passages.

### Example 11. Bio-Similar Matrix Environments

The following experiments suggest that HIPEC lines of the described invention can be used as model systems for the evaluation of various materials and conditions, including, but not limited to, therapeutic agents, cellular toxicity, and disease states.

### Example 11.1. Monolayer Formation by Isolated Human Gastrointestinal Epithelial Stem Cell Derived Epithelial Cells on a Biosimilar Matrix

A monolayer of gastrointestinal epithelial stem cell-like progenitor cells was cultivated on a bio-similar matrix environment (BSME) formed from the human mucosal tissue derived from a human gastrointestinal segment. Briefly, cells were grown on a transwell membrane coated with biosimilar matrix as described in **Example 1.**

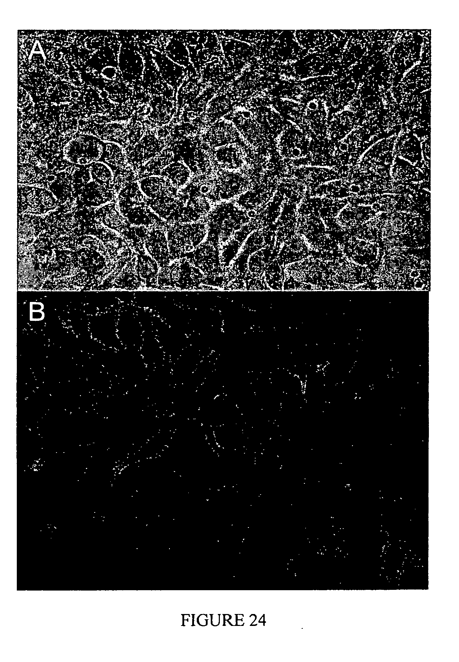

**Figure 24** shows micrographs of the cell monolayer. Immunofluorescence staining utilizing anti-human E-cadherein (Transduction Laboratories) (green) showed expression of cell:cell junction formation protein E-cadherin. DAPI, or 4',6-diamidino-2-phenyl indole, is a fluorescent stain that binds simply to DNA. DAPI staining (blue) shows the nucleus of the cells in the monolayer. These results indicate the formation of (i) a cell monolayer on the biosimilar matrix environment, and (ii) cell:cell junctions.

### Example 11.2. Assessment of Cellular Toxicity

HGISC-derived primary epithelial cell lines were utilized to provide a system to assess cellular toxicity of a therapeutic agent.

Briefly, HGISC-derived primary epithelial cell lines (A2J1 and SOJ2) and the malignant colonic adenocarcinoma cell line (HT29) were cultured, as described above in Examples 1A and 1B in medium containing different cytotoxic levels of the irinotecan metabolite 7-ethyl-10-hydroxy-20(S)-camptothecin (SN-38). Irinotecan is a chemotherapeutic agent mainly used in colon cancer, and the SN-38 metabolite is 200-fold more active than irinotecan itself. Each cell culture was assessed for viability utilizing the MTT assay.

Yellow MTT (3-(4,5-Dimethylthiazol-2-yl)-2,5-diphenyltetrazolium bromide, a tetrazole) is reduced to purple formazan in living cells. A solubilization solution (usually either dimethyl sulfoxide, an acidified ethanol solution, or a solution of the detergent sodium dodecyl sulfate in diluted hydrochloric acid) is added to dissolve the insoluble purple formazan product into a colored solution. The absorbance of this colored solution may be quantified by measuring at a certain wavelength (usually between 500 and 600 nm) using a spectrophotometer. The absorption maximum is dependent on the solvent employed. The MTT assay may be used to determine cytotoxicity of materials, since those materials that result in cell toxicity, and therefore metabolic dysfunction, yield decreased performance in the assay.

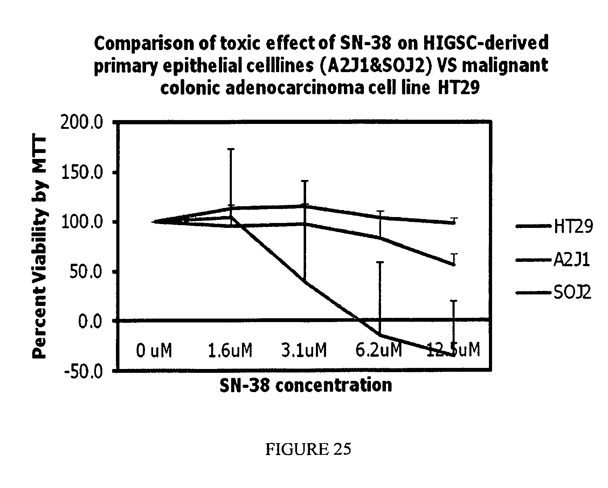

**Figure 25** shows the percent viability of the cultures as determined by MTT plotted against SN-38 concentration. The results indicate that the percent viability of the HT29 cells is unaffected by increasing amounts of SN-38, the percent viability of the SOJ2 cells remains above 50%, and the percent viability of the A2J1 cells decrease to 0%. Thus, it is concluded that the primary cells (which represent the normal portion of the body) are much more susceptible to a therapeutic agent (SN-38) than those cells (HT29) typically affected by the therapeutic agent (SN-38). This example shows that the human gastrointestinal stem cell (HGISC)-derived primary epithelial cell lines are useful for assessing cellular toxicity/safety of a therapeutic agent.

### Example 11.3. Therapeutic Agents: 5-aminosalicyclic acid

The anti-inflammatory therapeutic agent 5-aminosalicyclic acid (5-ASA) is used to treat inflammatory bowel syndrome (IBS) and ulcerative colitis. When administered *in vivo,* it is delivered to the small and large intestine where it is active against the inflammation seen in IBS. In the inflamed intestinal mucosa of ulcerative colitis, the levels of several pro-inflammatory cytokines (IL-1β, IL-6, IL-8, interferon-γ and TNF-α) are elevated.

Human gastrointestinal epithelial stem cell-like progenitor cells were isolated from a normal segment of the large intestine and a segment of the large intestine in a diseased state (such as a segment having ulcerative colitis). Epithelial monolayers were grown from these progenitor crypt cells on the appropriate BSME as described above.

IFN-γ is a cytokine produced predominantly by natural killer T cells as part of the innate immune response, and by CD4 and CD8 cytokine T lymphocyte effector T-cells once antigen specific immunity develops. It also is produced by Th1 cells, NK cells, and CD8 cytotoxic T cells.

Bacterial lipopolysaccharides ("LPS"), also referred to as lipoglycan, are large molecules found in the outer membrane of Gram negative bacteria, which elicit strong immune responses.

Interleukin-8 ("IL-8") is a chemokine produced by macrophages and other cell types such as epithelial cells and is a mediator of the immunoreaction in the innate immune system response. It serves as a chemical signal that attracts neutrophils at the site of inflammation.

The effects of 5-ASA on spontaneous, LPS, or IFN-γ stimulated IL-8 production were assessed on HIPEC cells cultured in the presence or absence of either LPS or IFN-γ and 5-ASA (1-10 µg/ml). The level of IL-8 production was measured by ELISA.

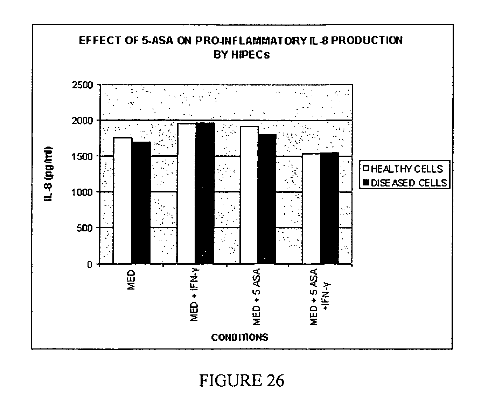

**Figure 26** shows a bar graph illustrating IL-8 (pg/ml) production by HIPECs as a function of medium conditions. As shown in **Figure 26****,** HIPECs from both normal controls and from patients with IBS showed high levels of spontaneous IL-8 production that was moderately enhanced by LPS and IFN-γ stimulation. The addition of 5-ASA in the culture medium of both normal and IBS HIPECs suppressed IL-8 production. Without being limited by theory, it appears that the anti-inflammatory action of 5-ASA is mediated by inhibition of IFN-γ stimulated IL-8 production in intestinal epithelial cells.

### Example 11.4. Determination of the metabolic byproducts of a therapeutic agent

According to this example, gastrointestinal epithelial stem-cell-like progenitor cells are isolated from gastrointestinal mucosal tissue segments as described in Example 1 A and 1B. A corresponding BSME is formed for each gastrointestinal tissue segment as described in Example 2. Each BSME is supplemented with corresponding Gastrointestinal mucosal tissue derived growth supporting factors (MTD-GSFs) appropriate for each gastrointestinal tissue segment. The gastrointestinal epithelial stem-cell-like progenitor cells are seeded onto their corresponding BSMEs and incubated to allow for the formation of an epithelial monolayer of gastrointestinal epithelial stem-cell-like progenitor cells. The BSMEs and accompanying epithelial monolayers represent each segment of the human gastrointestinal tract. Metabolites of a therapeutic agent in each segment or in serial segments are detected.

### Example 11.5. Identification of therapeutic targets useful for treating diseases of the gastrointestinal tract

According to this example, gastrointestinal epithelial stem-cell-like progenitor cells are prepared from normal tissue, from normal tissue in a diseased state (meaning normal tissue obtained from a non-diseased section of a diseased tissue), and normal tissue in a diseased state resulting from chemical and/or biological induction (meaning normal tissue obtained from a non-diseased section of a diseased tissue where the disease was induced in vitro) from gastrointestinal mucosal tissue segment. A corresponding BSME is formed for each gastrointestinal tissue segments. Each BSME is supplemented with corresponding MTD-GSFs appropriate for each gastrointestinal tissue segment. The gastrointestinal epithelial stem-cell-like progenitor cells are seeded onto their corresponding BSMEs and incubated to allow for the formation of an epithelial monolayer of gastrointestinal epithelial stem-cell-like progenitor cells.

The cellular matrix and morphology of each monolayer is monitored and analyzed for differences. Profiles of the nucleic acids, proteins, and other expressed molecules within each of the gastrointestinal epithelial stem-cell-like progenitor cells are generated via bioanalytical assays, including, but not limited to, ELISA, NMR, LC-MS, or HPLC.

Detected differences represent potential therapeutic targets, which will be further isolated and purified. Since each of the epithelial monolayers is a physiological representation of the corresponding gastrointestinal tissue segment, a direct correlation may be made between the identified therapeutic targets and *in vivo* gastrointestinal tissue.

Additionally, a therapeutic agent under study is administered to each of the epithelial monolayers, and profiles of nucleic acids, proteins and other expressed molecules as well as electrical resistance measurements are obtained. Profiles generated before and after the administration of the therapeutic agent are compared to identify potentially useful therapeutic targets.

## Claims

1. A system to determine the bioavailability of a therapeutic agent in at least one gastrointestinal segment of a human, said system comprising:
- differentiable gastrointestinal segment-specific human epithelial stem-cell-like progenitor cells isolated from at least one human mucosal tissue derived from at least one human gastrointestinal segment; and
- a growth substrate upon which the progenitor cells are grown, said growth substrate comprising a segment specific bio-similar matrix environment (BSME) formed from the segment of the gastrointestinal human mucosal tissue.

2. The system according to claim 1, wherein the gastrointestinal segment-specific human epithelial stem cell-like progenitor cells are cultivated on said growth substrate.

3. The system according to claim 1, wherein the segment is selected from the group consisting of: a stomach segment, a jejunum segment, an ileum segment, a duodenum segment, an ascending colon segment, a transverse colon segment, a sigmoid colon segment and a rectum segment.

4. The system according to claim 1, wherein the differentiable gastrointestinal segment-specific human epithelial stem-cell-like progenitor cell with differentiate into a mature cell phenotype.

5. The system according to claim 4, wherein the mature cell phenotype is selected from the group consisting of: a columnar epithelial cell, a Paneth cell, a goblet cell, an enteroendocrine chromaffin cell and a neuronal cell type.

6. The system according to claim 1, wherein the differentiable gastrointestinal segment-specific human epithelial stem-cell-like progenitor cell is a mesenchymal cell.

7. The system according to claim 1 or 2, wherein the bio-similar matrix environment formed from the at least one mucosal tissue derived from the stomach is serially connected to the bio-similar matrix environment formed from the at least one mucosal tissue derived from the jejunum, which is serially connected to the bio-similar matrix environment formed from the at least one mucosal tissue derived from the ileum, which is serially connected to the bio-similar matrix environment formed from the at least one mucosal tissue derived from the duodenum, which is serially connected to the bio-similar matrix environment formed from the at least one mucosal tissue derived from the ascending colon segment, which is serially connected to the bio-similar matrix environment formed from the at least one mucosal tissue derived from the transverse colon segment, which is serially connected to the bio-similar matrix environment formed from the at least one mucosal tissue derived from the sigmoid colon segment, which is serially connected to the bio-similar matrix environment formed from the at least one mucosal tissue derived from the rectum to form an *in vitro* model of the human gastrointestinal tract.

8. The system according to claim 1, wherein the differentiable gastrointestinal segment-specific human epithelial stem cell-like progenitor cell has at least a β-1-integrin⁽⁺⁾cytokeratin⁽⁺⁾ phenotype.

9. The system according to claim 8, wherein the differentiable gastrointestinal segment-specific human epithelial stem cell-like progenitor cell has a phenotype of cytokeratin(+), β-1-integrin(+), defensin-5(+), trefoil factor-3(+), mucin-2(+), chomogranin-A(+), intestinal alkaline phosphatase(+), lysozyme(+).

10. The system according to claim 1 or 2 for use to assess at least one parameter of permeability of the therapeutic agent, to assess absorption of the therapeutic agent, to assess uptake of the therapeutic agent, to assess cellular toxicity of the therapeutic agent, to assess transepithelial electrical resistance, to determine variations in DNA and/or RNA characteristics produced in response to the therapeutic agent, or to determine segment-specific metabolic byproducts of the therapeutic agent.

## Patentansprüche

1. System zum Bestimmen der Bioverfügbarkeit eines therapeutischen Mittels in mindestens einem Gastrointestinalsegment eines Menschen, wobei das System umfasst:
- differenzierbare gastrointestinalsegmentspezifische, humane, epitheliale, stammzellenartige Vorläuferzellen, die aus mindestens einem menschlichen Schleimhautgewebe isoliert sind, das aus mindestens einem humanen Gastrointestinalsegment stammt; und
- ein Wachstumssubstrat, auf dem die Vorläuferzellen aufwachsen, wobei das Wachstumssubstrat ein segmentspezifisches, biosimilares Mutterge-webemilieu (BSME - *bio-similar matrix environment*) umfasst, das aus dem Segment des gastrointestinalen humanen Schleimhautgewebes gebildet ist.

2. System nach Anspruch 1, wobei die gastrointestinalsegmentspezifischen, hu-manen, epithelialen, stammzellenartigen Vorläuferzellen auf dem Wachstumssubstrat kultiviert werden.

3. System nach Anspruch 1, wobei das Segment aus der Gruppe ausgewählt ist, die besteht aus: einem Magensegment, einem Leerdarmsegment, einem Krummdarmsegment, einem Zwölffingerdarmsegment, einem Segment des aufsteigenden Kolons, einem Querkolonsegment, einem Sigmadarmsegment und einem Enddarmsegment.

4. System nach Anspruch 1, wobei sich die differenzierbare gastrointestinalseg-mentspezifische, humane, epitheliale, stammzellenartige Vorläuferzelle in einen Phänotyp einer reifen Zelle differenzieren wird.

5. System nach Anspruch 4, wobei der Phänotyp einer reifen Zelle aus der Gruppe ausgewählt ist, die besteht aus: einem zylindrischen Epithelzell-, einem Paneth-Zell-, einem Becherzell-, einem enteroendokrinen chromaffinen Zell- und einem neuronalen Zelltypus.

6. System nach Anspruch 1, wobei es sich bei der differenzierbaren gastrointestinalsegmentspezifischen, humanen, epithelialen, stammzellenartigen Vorläuferzelle um eine mesenchymale Zelle handelt.

7. System nach Anspruch 1 oder 2, wobei das biosimilare Muttergewebemilieu, das aus dem mindestens einen, aus dem Magen stammenden Schleimhautgewebe gebildet ist, seriell an das biosimilare Muttergewebemilieu angeschlossen wird, das aus dem mindestens einen Schleimhautgewebe gebildet ist, das vom Leerdarm stammt, das seriell an das biosimilare Muttergewebemilieu angeschlossen wird, das aus dem mindestens einen Schleimhautgewebe gebildet ist, das vom Krummdarm stammt, das seriell an das biosimilare Muttergewebemilieu angeschlossen wird, das aus dem mindestens einen Schleimhautgewebe gebildet ist, das vom Zwölffingerdarm stammt, das seriell an das biosimilare Muttergewebemilieu angeschlossen wird, das aus dem mindestens einen Schleimhautgewebe gebildet ist, das vom aufsteigenden Kolonsegment stammt, das seriell an das biosimilare Muttergewebemilieu angeschlossen wird, das aus dem mindestens einen Schleimhautgewebe gebildet ist, das vom Querkolonsegment stammt, das seriell an das biosimilare Muttergewebemilieu angeschlossen wird, das aus dem mindestens einen Schleimhautgewebe gebildet ist, das vom Sigmadarmsegment stammt, das seriell an das biosimilare Muttergewebemilieu angeschlossen wird, das aus dem mindestens einen Schleimhautgewebe gebildet ist, das vom Enddarm stammt, um ein *in vitro*-Modell des humanen Gastrointestinaltrakts zu bilden.

8. System nach Anspruch 1, wobei die differenzierbare gastrointestinalsegmentspezifische, humane, epitheliale, stammzellenartige Vorläuferzelle zumindest einen β-1-Integrin⁽⁺⁾-Cytokeratin⁽⁺⁾-Phänotyp aufweist.

9. System nach Anspruch 8, wobei die differenzierbare gastrointestinalsegmentspezifische, humane, epitheliale, stammzellenartige Vorläuferzelle einen Phänotyp von Cytokeratin(+), β-1-Integrin(+), Defensin-5(+), Trefoil Faktor-3(+), Mucin-2(+), Chomogranin-A(+), intestinale alkalische Phosphatase(+), Lysozym(+) aufweist.

10. System nach Anspruch 1 oder 2 zur Verwendung, um mindestens einen Permeabilitätsparameter des therapeutischen Mittels zu bewerten, um eine Absorption des therapeutischen Mittels zu bewerten, um eine Aufnahme des therapeutischen Mittels zu bewerten, um eine zelluläre Toxizität des therapeutischen Mittels zu bewerten, um einen transepithelialen elektrischen Widerstand zu bewerten, um Veränderungen bei DNA- und/oder RNA-Charakteristika zu bestimmen, die im Ansprechen auf das therapeutische Mittel hervorgerufen werden, oder um segmentspezifische metabolische Nebenprodukte des therapeutischen Mittels zu bestimmen.

## Revendications

1. Système pour déterminer la biodisponibilité d'un agent thérapeutique dans au moins un segment gastro-intestinal d'un être humain, ledit système comportant :
- des cellules progénitrices différentiables analogues à des cellules souches épithéliales humaines spécifiques à un segment gastro-intestinal, isolées à partir d'au moins un tissu mucosal humain provenant d'au moins un segment gastro-intestinal humain ; et
- un substrat de croissance sur lequel les cellules progénitrices sont mises à croître, ledit substrat de croissance comportant un environnement matriciel bio-similaire spécifique à un segment (BSME) formé à partir du segment du tissu mucosal humain gastro-intestinal.

2. Système selon la revendication 1, dans lequel les cellules progénitrices analogues des cellules souches épithéliales humaines spécifiques à un segment gastro-intestinal sont mises en culture sur ledit substrat de croissance.

3. Système selon la revendication 1, dans lequel le segment est sélectionné dans un groupe comprenant : un segment d'estomac, un segment de jéjunum, un segment d'iléon, un segment de duodénum, un segment de côlon ascendant, un segment de colon transversal, un segment de colon sigmoïde et un segment de rectum.

4. Système selon la revendication 1, dans lequel la cellule progénitrice différentiable analogue à une cellule souche épithéliale humains spécifique à un segment gastro-intestinal va se différencier en un phénotype cellulaire mature.

5. Système selon la revendication 4, dans lequel le phénotype cellulaire mature est sélectionné dans le groupe comprenant : une cellule épithéliale colonnaire, une cellule de Paneth, une cellule caliciforme, une cellule chromaffine entéroendocrine et un type de cellules neuronales.

6. Système selon la revendication 1, dans lequel la cellule progénitrice différentiable analogue à une cellule souche épithéliale humaine spécifique à un segment gastro-intestinal est une cellule mésenchymateuse.

7. Système selon la revendication 1 ou 2, dans lequel l'environnement matriciel bio-similaire formé à partir du au moins un tissu mucosal provenant de l'estomac est relié en série à l'environnement matriciel bio-similaire formé à partir du au moins un tissu mucosal provenant du jéjunum, qui est relié en série à l'environnement matriciel bio-similaire formé à partir du au moins un tissu mucosal provenant de l'iléon, qui est relié en série à l'environnement matriciel bio-similaire formé à partir du au moins un tissu mucosal provenant du duodénum, qui est relié en série à l'environnement matriciel bio-similaire formé à partir du au moins un tissu mucosal provenant du segment de colon ascendant, qui est relié en série à l'environnement matriciel bio-similaire formé à partir du au moins un tissu mucosal provenant du segment de colon transverse, qui est relié en série à l'environnement matriciel bio-similaire formé à partir du au moins un tissu mucosal provenant du segment de colon sigmoïde, qui est relié en série à l'environnement matriciel bio-similaire formé à partir du au moins un tissu causal provenant du rectum, pour former un modèle *in vivo* du conduit gastro-intestinal humain.

8. Système selon la revendication 1, dans lequel la cellule progénitrice différentiable analogue à une cellule souche épithéliale humaine spécifique à un segment gastro-intestinal a au moins un phénotype β-1-intégrine⁽⁺⁾cytokératine⁽⁺⁾.

9. Système selon la revendication 8, la cellule progénitrice différentiable analogue à une cellule souche épithéliale humaine spécifique à un segment gastro-intestinal a un phénotype de cytokératine (+), β-1-intégrine (+), défensine-5(+), facteur en feuille de trèfle(+), mucine-2(+), chomogranine-A(+), phosphatase alcaline intestinale (+), et lysozyme (+).

10. Système selon la revendication 1 ou 2, pour une utilisation afin d'évaluer au moins un paramètre de perméabilité de l'agent thérapeutique, pour évaluer l'absorption de l'agent thérapeutique, pour évaluer la prise de l'agent thérapeutique, pour évaluer la toxicité cellulaire de l'agent thérapeutique, pour évaluer la résistance électrique transépithéliale, pour déterminer des variations de caractéristiques d'ADN et/ou ARN produit(s) en réponse à l'agent thérapeutique, ou pour déterminer des produits dérivés métaboliques spécifiques à un segment de l'agent thérapeutique.


### SEQUENCE LISTING

<110> Alphagene Bioscience, Inc.
<120> Drug Discovery Methods Involving a Preclinical, In Vitro Isolated Gastrointestinal Epithelial Stem Cell-like Progenitor Cell System
<130> 117471.010100
<150> US 61/047,296
   <151> 2008-04-23
<160> 20
<170> PatentIn version 3.5
<210> 1
   <211> 20
   <212> DNA
   <213> Unknown
<220>
   <223> MAMMALIAN
<400> 1
   atgcctgtga tttgtgggcc 20
<210> 2
   <211> 25
   <212> DNA
   <213> Unknown
<220>
   <223> MAMMALIAN
<400> 2
   ctcatcttca cacgtcttca ggttg 25
<210> 3
   <211> 24
   <212> DNA
   <213> Unknown
<220>
   <223> MAMMALIAN
<400> 3
   caaccagcag tttgcaggtg gctg 24
<210> 4
   <211> 26
   <212> DNA
   <213> Unknown
<220>
   <223> MAMMALIAN
<400> 4
   gaaccctcac ttgcatttgg acagag 26
<210> 5
   <211> 24
   <212> DNA
   <213> Unknown
<220>
   <223> MAMMALIAN
<400> 5
   cgggacacct ggcttcggat ttcg 24
<210> 6
   <211> 24
   <212> DNA
   <213> Unknown
<220>
   <223> MAMMALIAN
<400> 6
   cttgtaagaa cataaacaca ccag 24
<210> 7
   <211> 25
   <212> DNA
   <213> Unknown
<220>
   <223> MAMMALIAN
<400> 7
   catcaaagct ctgcagaaag aactc 25
<210> 8
   <211> 25
   <212> DNA
   <213> Unknown
<220>
   <223> MAMMALIAN
<400> 8
   ctgcttgatc gcttgccctt ctggc 25
<210> 9
   <211> 24
   <212> DNA
   <213> Unknown
<220>
   <223> MAMMALIAN
<400> 9
   cttgtaagaa cataaacaca ccag 24
<210> 10
   <211> 24
   <212> DNA
   <213> Unknown
<220>
   <223> MAMMALIAN
<400> 10
   ctggaggctt agggaccaag gctg 24
<210> 11
   <211> 23
   <212> DNA
   <213> Unknown
<220>
   <223> MAMMALIAN
<400> 11
   catgagtcag tgaacaggga atg 23
<210> 12
   <211> 24
   <212> DNA
   <213> Unknown
<220>
   <223> MAMMALIAN
<400> 12
   ggtttctgct ttgcatatct cctg 24
<210> 13
   <211> 27
   <212> DNA
   <213> Unknown
<220>
   <223> MAMMALIAN
<400> 13
   cataatagaa tgtctacatt ccttctg 27
<210> 14
   <211> 24
   <212> DNA
   <213> Unknown
<220>
   <223> MAMMALIAN
<400> 14
   ggaagtggac cattccttct ccag 24
<210> 15
   <211> 25
   <212> DNA
   <213> Unknown
<220>
   <223> MAMMALIAN
<400> 15
   gatctgtctt acaacctatt agaag 25
<210> 16
   <211> 25
   <212> DNA
   <213> Unknown
<220>
   <223> MAMMALIAN
<400> 16
   cttcaaggtc acgttcatct tgagc 25
<210> 17
   <211> 28
   <212> DNA
   <213> Unknown
<220>
   <223> MAMMALIAN
<400> 17
   caaaagtctt taccaataat atttagag 28
<210> 18
   <211> 26
   <212> DNA
   <213> Unknown
<220>
   <223> MAMMALIAN
<400> 18
   gccgaatctt ttaaaataca actacg 26
<210> 19
   <211> 26
   <212> DNA
   <213> Unknown
<220>
   <223> MAMMALIAN
<400> 19
   taaaagttct agtggtacgg taggag 26
<210> 20
   <211> 26
   <212> DNA
   <213> Unknown
<220>
   <223> MAMMALIAN
<400> 20
   gccgaatctt ttaaaataca actacg 26